(19) Europäisches Patentamt
European Patent Office
Office européen des brevets

(11) Veröffentlichungsnummer : **0 531 300 B1**

# (12) EUROPÄISCHE PATENTSCHRIFT

(45) Veröffentlichungstag der Patentschrift : **08.06.94 Patentblatt 94/23**

(51) Int. Cl.$^5$ : **C12N 15/12,** C12P 21/08, A61K 37/02, C07K 13/00, G01N 33/574

(21) Anmeldenummer : **91906994.8**

(22) Anmeldetag : **30.03.91**

(86) Internationale Anmeldenummer : **PCT/EP91/00614**

(87) Internationale Veröffentlichungsnummer : **WO 91/17248 14.11.91 Gazette 91/26**

## (54) VARIANTE CD44-OBERFLÄCHENPROTEINE, DIESE KODIERENDE DNA-SEQUENZEN, ANTIKÖRPER GEGEN DIESE PROTEINE SOWIE IHRE VERWENDUNG IN DER DIAGNOSTIK UND THERAPIE.

(30) Priorität : **07.05.90 DE 4014510**

(43) Veröffentlichungstag der Anmeldung : **17.03.93 Patentblatt 93/11**

(45) Bekanntmachung des Hinweises auf die Patenterteilung : **08.06.94 Patentblatt 94/23**

(84) Benannte Vertragsstaaten : **AT BE CH DE DK FR GB GR IT LI LU NL SE**

(56) Entgegenhaltungen :
**Cancer Research, vol. 49, 1989, S. Matzku et al., S. 1294-1299**
**Anticancer Research, Band 8, Nr. 6, Dezember 1988, M. Birnbaum et al., S. 1185-1192**

(73) Patentinhaber : **Kernforschungszentrum Karlsruhe GmbH**
**Weberstrasse 5**
**D-76133 Karlsruhe (DE)**
Patentinhaber : **UNIVERSITÄT KARLSRUHE(TECHNISCHE HOCHSCHULE)**
**Kaiserstrasse 12**
**D-76131 Karlsruhe (DE)**
Patentinhaber : **Deutsches Krebsforschungszentrum**
**Im Neuenheimer Feld 280**
**D-69120 Heidelberg (DE)**

(72) Erfinder : **HERRLICH, Peter**
**Vogelsang 8**
**D-Karlsruhe 41 (DE)**
Erfinder : **PONTA, Helmut**
**Blankenlocherstrasse 12**
**D-7515 Linkenheim (DE)**
Erfinder : **GÜNTHERT, Ursula**
**Gerwigstrasse 40**
**D-7500 Karlsruhe 1 (DE)**
Erfinder : **MATZKU, Siegfried**
**Schillerstrasse 9**
**D-6901 Wiesenbach (DE)**
Erfinder : **WENZEL, Achim**
**Handschuhsheimer Landstr. 86**
**D-6900 Heidelberg (DE)**

(74) Vertreter : **Grussdorf, Jürgen, Dr. et al**
**Patentanwälte Zellentin & Partner**
**Rubensstrasse 30**
**D-67061 Ludwigshafen (DE)**

EP 0 531 300 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft variante CD44-Oberflächenproteine, Antikörper gegen die variante Determinante dieser Proteine sowie Verfahren zu ihrer Herstellung, außerdem die DNA Sequenzen, die für diese varianten Proteinstücke kodieren, sowie die Verwendung dieser Proteine oder Teile davon und die gegen sie gerichteten Antikörper zur Diagnostik und Therapie von Tumormetastasen.

Die Fähigkeit zu metastasieren bildet die eigentliche lebensbedrohliche Eigenschaft maligner Tumorzellen. Die ursprünglichen Primärtumorzellen erwerben diese Eigenschaft vermutlich durch eine ganze Serie von Veränderungen im Verlauf der Tumorprogression. Als Ergebnis dieses Prozesses lösen sich Krebszellvarianten ständig von der primären Tumormasse ab, durchdringen die extrazelluläre Matrix und wandern in das lymphatische System oder die Blutzirkulation ein. Oft aneinander klebend, werden die metastasierenden Tumorzellen im Blut- oder Lymphsystem transportiert, verlassen das Gefäßsystem an anderen Stellen, um dort in Sekundärgewebe einzudringen und Tochtertumoren zu bilden (Übersichten von Hart et al., 1989; Nicolson, 1987). Die Bildung von Metastasen erfordert eine ganze Reihe von Interaktionen der Tumorzellen mit interzellulärer Matrix oder anderen Zellen. Fast alle dieser Interaktionen benötigen Zelloberflächenkomponenten wie z.B. die Rezeptoren für Matrix und Lamina, oberflächengebundene proteolytische Enzyme, sowie Zelladhäsionsmoleküle unter Einschluß solcher, die organspezifische Adhäsion und damit Organpräferenz der Metastasierung bedingen, außerdem Wachstumsfaktoren und Wachstumsfaktorrezeptoren.

Es ist bekannt, daß sich die Membranproteine nichtmetastasierender und metastasierender Tumorzellen des BSp73 RattenTumors unterscheiden, nachgewiesen durch Antikörperreaktion (Matzku et al., 1983 und 1989).

Es wurde nunmehr gefunden, daß die metastasierenden BSp73ASML Tumorzellen ein Oberflächenprotein enthalten, das zum Teil einem bekannten an der Lymphozytenadhäsion und Zell-Zell und Zell-Matrix Wechselwirkung beteiligten Glykoprotein entspricht (Bezeichnungen des normalen Glykoproteins beim Menschen: CD44, Hermes-I, bei der Maus: Ppg-I und bei der Ratte: HEBFln). Von diesen bekannten Sequenzen unterscheidet sich das neue variante CD44-Oberflächenprotein jedoch durch einen extrazellulären Bereich (EZB) von 154 Aminosäuren, der zwischen der 220. und der 237. Aminosäure der menschlichen CD44-Sequenz eingeschoben ist (bzw. 224. und 239. Aminosäure der Maus-Sequenz). Dieses neue Glykoprotein scheint eine wichtige Rolle für die Zell/Matrix bzw. Zell/Zellbindung bei der Metastasierung zu besitzen. Herstellung und Charakterisierung dieses Proteinbereichs (EZB) bilden daher eine der Aufgaben der vorliegenden Erfindung. Durch Immunisierung von Mäusen mit Membranproteinen, die aus BSp73ASML gewonnen wurden, wurden Milz-Zellen erzeugt, die Antikörper gegen den EZB des varianten CD44-Oberflächenproteins bilden. Nach der Methode von Köhler (1981) wurden diese durch Polyäthylenglykol mit Myelomzellen verschmolzen, um beständige Kulturen zu erzeugen. Durch Klonierung und Auswahl derjenigen Kulturen, die Antikörper produzieren, die mit BSp73ASML, nicht jedoch mit der nichtmetastasierenden Stammform BSp73AS reagieren und auch nicht mit anderen, nichttumorogenen Rattenzellen, lassen sich gegen den neuen Proteinteil EZB spezifische Antikörper gewinnen. Für die weitere Untersuchung wurde ein monoklonaler Antikörper ausgewählt,der die BSp73ASML Zellen im Immunfluoreszenztest besonders intensiv anfärbt, der die Bezeichnung erhielt mAbl.IASML (mAb: monoklonaler Antikörper).

Im Westernblot Test lassen sich in einem Proteinhydrolysat aus BSp73ASML Zellen 4 Proteinbanden mit Molekulargewichten von 120.000, 150.000, 180.000 und 200.000 mit mAbl.I.ASML bestimmen, während Extrakte aus Rattenfibroblastenzellen und nicht-metastasierenden Rattentumorzellen keine signifikate Reaktion ergeben. Es konnte noch nicht bestimmt werden, ob diese Größenunterschiede auf einer unterschiedlichen ursprünglichen Aminosäuresequenz oder auf einer verschieden starken nachträglichen Proteinmodifikation beruhen. In jedem Fall ist das von dem Antikörper erkannte Epitop in allen 4 Proteinspezies enthalten, nicht jedoch in den zur Kontrolle dienenden Proteinen aus den nichtmetastasierenden BSp73AS-Zellen oder aus normalen Rattenzellen.

**Isolierung von cDNA Sequenzen, die für den EZB des Oberflächenproteins kodieren.**

Der monoklonale Antikörper mAbl.IASML wurde verwendet, um die EZB-kodierenden cDNA-Sequenzen in einer bakteriellen Expressionsbibliothek zu entdecken. Die Bibliothek wurde mit Hilfe von PolyA+ RNA aus BSp73ASML Zellen und dem pEX Vektor System konstruiert (Stanley & Luzio, 1984). Die von den cDNA-Sequenzen kodierten Produkte werden als β-Galaktosidase-Fusionsproteine mit Hilfe der Antikörper aufgefunden. Ein so isolierter, für den monoklonalen Antikörper I.IASML positiver cDNA Klon mit dem Namen pEX34 trägt ein cDNA Sequenzstück von 167 Nukleotiden. Dieses cDNA-Stück wurde nun benutzt um eine größere cDNA Bibliothek im Vektor pSP65, wiederum aus BSp73ASML RNA, durchzumustern. Einer dieser damit isolierten Klone, pM66, diente dann dazu, mit Hilfe von sogenannter "Primer"-Verlängerung (Starter-Oligonukleotide) und der Polymerase-Ketten-Reaktion (PCR) den Gesamtlängen cDNA-Klon pMeta-I zu isolieren. Beweis der vollen Länge wurde erhalten mit Hilfe der Primerverlängerung (3207 Nukleotide). Die Kolinearität mit einer RNA aus dem BSp73ASML-Tumor wurde dokumentiert durch RNase und SI Schutzanalyse.

Die Isolierung der cDNAs mit Hilfe der Expression in Bakterien und dem Erkennen durch den Antikörper beweist, daß der Antikörper primäre Aminosäuresequenz im EZB des Oberflächenproteins erkennt.

**EZB-kodierende Messenger RNA wird exprimiert in BSp73ASML, aber nicht in BSp73AS-Zellen.**

Drei verschiedene Sequenzproben wurden aus den cDNA Klonen erzeugt, um spezifische Messenger-RNAs durch Hybridisierung nachzuweisen. Die Probe A überdeckt den cDNA-Bereich, der für das EZB kodiert (Positionen: 941-1108). Die Probe B repräsentiert Sequenzen zwischen den Positionen 1403-1572 und Probe C trägt Sequenzen vom Anfang bis zur Position 794 aus pMeta-l (Abb.I). Poly A+ RNA der BSp73 Tumorzellinien wurde elektrophoretisch aufgetrennt und mit Hilfe der RNA Transferhybridisierung analysiert. Vier der Zellinien, die nicht metastasieren, enthalten keine RNA, die zur Probe A homolog ist, während RNAs aus den metastasierenden Tumorzellen BSp73ASML stark reagieren. Die Probe A erkennt in dieser RNA-Präparation eine heterogene Mischung verschiedener RNA-Größen zwischen 2,2 und 3,3 kb, und eine größere RNA-Spezies von 4,9 kb. Die ausschließliche Expression der vom monoklonalen Antikörper I.IASML erkannten spezifischen Membranproteine in der metastasierenden Tumorzellvariante ist offensichtlich begründet durch die ausschließliche Expression der entsprechenden EZB-kodierenden Messenger-RNAs. Offensichtlich kann der vollständige cDNA-Klon pMeta-l mit 3,2 kb nicht alle Sequenzen dieser RNA-Spezies repräsentieren. Er kann nur eine Spezies aus dem heterogenen Gemisch an RNA-Größe darstellen. Die Proben B und C ergeben dasselbe Hybridisierungsmuster wie Probe A in der Auftrennung der BSp73ASML RNA, jedenfalls soweit man bei der Heterogenität feststellen kann, d.h. diese RNA-Spezies tragen Sequenzen, die komplementär zu allen drei Proben sind, A, B und C. Im Gegensatz zu Probe A erkennen Probe B und C auch Messenger-RNA-Spezies in den nicht-metastasierenden Zellinien. Die RNA Größen unterscheiden sich aber deutlich von denen in BSp-73ASML, es werden nämlich vier klar unterscheidbare Messenger-RNA-Spezies nachgewiesen, mit den Größen 1,5, 2,0, 2,9 und 4,3 kb. Obwohl diese RNA-Spezies im heterogenen Gemisch der RNAs aus BSp73ASML versteckt sein könnten, ist es doch sicher, daß sie nicht in der selben Menge in der BSp73ASML existieren. Entscheidend ist, daß RNA Sequenzen mit Komplementarität zu Probe A offensichtlich vollkommen abwesend sind in den nicht-metastasierenden Zellen. Wir können deshalb vorsichtig schließen, daß die Sequenzen der Proben A, B und C in denselben RNAs im metastasierenden Tumor enthalten sind, und zwar in einer Weise, als ob Probe-A-Sequenzen in die B-C-positiven RNAs hineingespleißt wären und als ob dieser alternative Spleiß-Vorgang nur in der metastasierenden Zellinie vorkäme.

Um die Kolinearität zwischen RNA und cDNA zu beweisen und um die Verschiedenheit der RNAs zwischen den BSp73AS und den BSp73ASML Zellen zu analysieren, wurden SI-Nuklease und RNase Schutzanalysen durchgeführt. Die geschützten DNA- oder RNA-Fragmente können nur kleiner sein als die Gesamtlänge, weil die 5 Enden Vektor-Sequenzen enthalten, die nicht mit den RNAs aus den Tumorzellen hybridisieren können. Wir betrachten zuerst den Übergang auf der 3′ Seite: den Übergang von Sequenzen mit Homologie zur Probe A nach solchen zur Probe B. Beide Techniken zeigen eine einzige RNA-Spezies in BSp73AS Zellen an, die mit den Proben über einen weiten Bereich kolinear ist. Weiter 5′ davon unterscheiden sich cDNA- bzw. RNA-Probe, die ja RNA-Sequenzen aus BSp73ASML entsprechen, von der RNA aus den BSp73AS-Zellen. Die RNAs von BSp73ASML enthalten vor allem Sequenzen, die größere Fragmente der Proben schützen. Die größten Fragmente entsprechen der vollen Länge der DNA- bzw. RNA-Stücke, die für die Protektionsanalyse angeboten wurden. Auch kleinere Fragmente sind nachweisbar. Da die RNA-Transfer-Hybridisierungen ja ein heterogenes Gemisch verschiedener Größen von RNAs aufgedeckt haben, ist es möglich, daß diese kleineren geschützten Fragmente RNA-Spezies anzeigen, die von der cDNA anderswo divergieren, d. h. an Stellen zwischen dem vorhin nachgewiesenen Divergenzpunkt und dem 5′ Ende der angebotenen Proben. Diese RNA-Spezies sind ebenso nicht nachweisbar in den BSp73AS-Zellen.

Betrachten wir nun den Punkt der Divergenz auf der 5′Seite, also den Übergang von Sequenzen, die mit der Probe C hybridisieren, zu denen, die mit der Probe A, also der EBZ-kodierenden Sequenz hybridisieren. Die Analyse ergibt entsprechende Resultate für den 5′ Bruchpunkt. RNAs aus BSp73AS Zellen können die angebotenen Proben nur über einen kleineren Bereich schützen. Messenger RNAs aus BSp73ASML Zellen schützen längere Fragmente. Sie sind nämlich kolinear über die gesamte Länge der angebotenen Probe. Man kann also schließen, daß der cDNA Klon pMeta-l Sequenzen repräsentiert, die in den metastasierenden Tumorzellen BSp73ASML ausgeprägt werden. Die 3′ und 5′ Bereiche werden auch in RNAs aus BSp73AS Zellen gefunden. Die EZB-kodierenden Sequenzen mit den definierten Übergängen, die mit Hilfe dieser oben geschilderten Techniken kartiert werden können, zeigen an, daß hier ein alternativer Spleißmechanismus für die RNA-Bildung vorliegen muß. Die 5′ und 3′ Bruchpunkte der Übergänge zu den EZB-Sequenzen sind in Abb.I durch Pfeile markiert.

**Der monoklonale Antikörper I.IASML identifiziert eine variante Form des Glykoproteins CD44.**

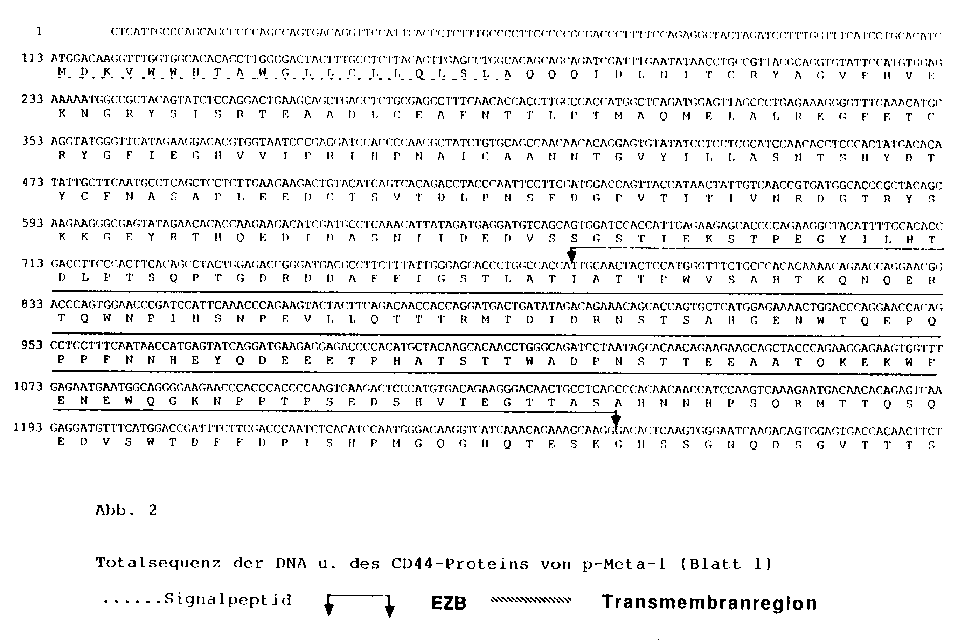

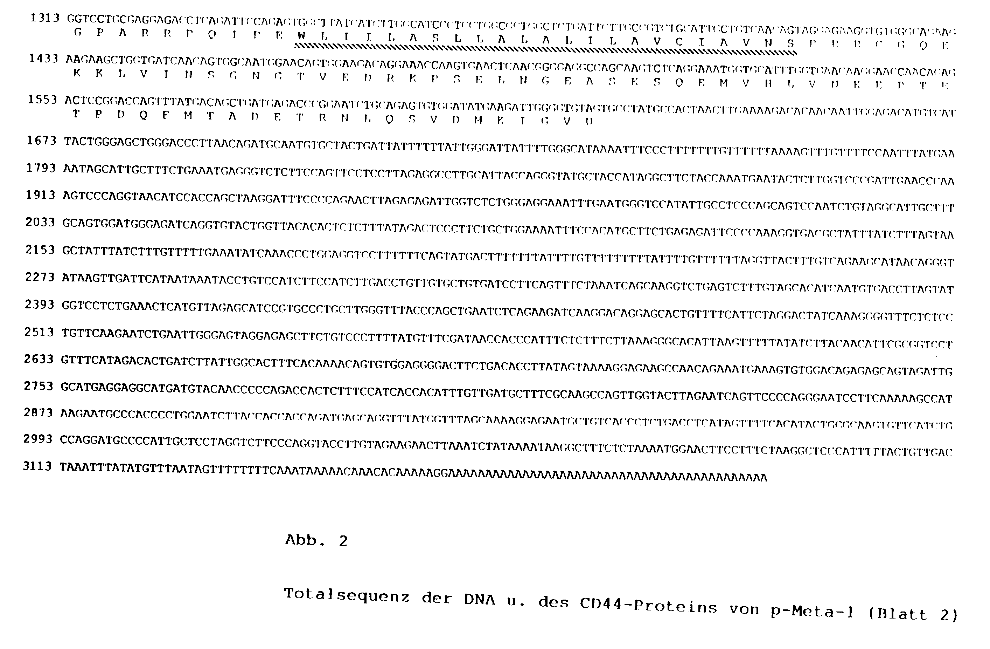

Um strukturelle Informationen über das Oberflächenprotein zu erhalten, sind alle geklonten cDNA Moleküle sequenziert worden. Die Nukleodid-Sequenz des Gesamtlängen-Klons pMeta-l und die davon abgeleiteten Aminosäuresequenzen sind in Abbildung 2 gezeigt. Der Gesamtlängen-cDNA-Klon überspannt 3207 Nukleotide. Der 3' Terminus trägt ein PolyA Ende, zwei zusätzliche Polyadenylierungssignale liegen bei den Positionen 2288 und 1743. Das erste ATG Kodon folgt einer Konsens-Initiationssequenz und eröffnet einen Leserahmen von 1509 Nukleotiden, entsprechend 503 Aminosäuren. Wie man für ein membranständiges Protein vermuten sollte, sind die ersten 21 Aminosäuren hydrophob und stellen ein Signalpeptid dar. Kein Teil dieser Sequenzen ist bisher in den Datenbasen zu finden. Wir fanden jedoch Sequenzhomologie zu kürzlich publizierten Daten über den LymphozytenHoming-Rezeptor CD44 (bzw. Pgp-I) (Idzerda et al., 1989; Goldstein et al., 1989; Stamenkovic et al., 1989; Nottenburg et al., 1989; Zhou et al., 1989). Die Homologien sind strikt beschränkt auf die 5' und 3' Teile der cDNA mit Einschluß nicht-translatierter Regionen und sie enden an den schon oben erwähnten Punkten der Divergenz zwischen den BSp73AS- und den BSp73ASML RNA-Sequenzen. Die gesamte Stecke zwischen den Diverenzpunkten (in der Abbildung 2 durch Farbmarkierung gekennzeichnet), also die ganze Strecke der metastasen-spezifischen EZB-kodierenden Sequenz, ist nicht repräsentiert in den Pgpl- oder CD44- Sequenzen. Das metastasen-spezifische Glykoprotein repräsentiert offensichtlich eine Variante der CD44 Glykoproteine. Es trägt nämlich eine zusätzliche extrazelluläre Domäne von 156 Aminosäuren und somit einen expandierten extrazellulären Bereich von 410 Aminosäuren (abzüglich 21 Aminosäuren Signalpeptid), gegenüber 270 Aminosäuren (ebenfalls abzüglich Signalpeptid) des unveränderten CD44 Glykoproteins. In den nicht-metastasierenden BSp73AS-Zellen jedoch werden die unveränderten Formen dieser CD44-Familie nachgewiesen. cDNA-Sequenzen dieser BSp73AS-RNAs sind ebenfalls kloniert worden und die Identität mit den metastasen-spezifischen Klonen <u>außerhalb</u> der Extradomäne ist nachgewiesen.

**Die Expression der varianten CD44 ist korreliert mit dem metastatischen Potential**

Um zu überprüfen, ob die Expression der varianten CD44 Glykoproteine ausnahmslos in den BSp73ASML Zellen erfolgt und ob sie in Zusammenhang mit dem metastatischen Potential dieser Zellen oder dem metastatischen Potential im allgemeinen steht, studierten wir eine andere Serie isogener Rattentumorzellinien, nämlich die Tumorzellinien des Mammakarzinom-Systems 13762 NF (Neri et al., 1982). Hier verglichen wir Zellinien, die vom parentalen Tumor hergeleitet wurden, nämlich die MTPa, MTC, MTF7 und MTA-Zellen (Gruppe 1), mit Zellinien, die aus Lymphknoten oder Lungenmetastasen etabliert wurden, nämlich MTLy, MTLn2, MTLn3 (Gruppe 2). Die Gruppe 1-Zellen exprimieren im wesentlichen das normale CD44 Muster, ähnlich dem der RNAs aus den BSp73AS Zellen, wenn man mit Probe B hybridisiert. Mit Probe A dagegen wird eine geringere Menge einer diffusen RNA Bande nachgewiesen, die etwa die Größe 2,5 kb hat. Die Gruppe 2-Zellen andererseits zeigen ein völlig verschiedenes RNA Muster. Beide Proben A und B hybridisieren mit größeren RNA Spezies. Die Größen ähneln denen, die in BSp73ASML nachgewiesen werden. Die Ähnlichkeit wird auch dokumentiert durch RNAse und SI Schutzanalysen. Aufgrund dieser Daten schließen wir, daß eine Veränderung im Spleißmuster der RNA und die Expression von variantem CD44 mit der Entstehung von Metastasen korreliert ist und daß das erworbene Muster in diesen metastasierenden Mammakarzinomzellen sehr denen entspricht, die wir oben schon für die metastasierende BSp73ASML Zellinie kennengelernt haben. Die hochmolekularen, durch den Antikörper erkannten Proteine entsprechen den beiden hochmolekularen Spezies an Proteinen, die in den BSp73ASML Extrakten nachgewiesen wurden. In dieser Mammatumorserie entdecken wir also ebenso eine metastasenspezifische Expression von RNA-Spezies und von hochmolekularen Proteinen. Daß in der Gruppe 1, also den sogenannten parentalen Zellinien überhaupt RNAs gefunden werden, die mit der Probe A, also der EBZ kodierenden Sequenz hybridisieren und daß wir auch eine schwache Färbung eines Proteins von 100.000 Daltons mit dem Antikörper sehen können, führen wir darauf zurück, daß die Gruppe 1-Zellen auch geringe Metastasierungsfähigkeit besitzen, ganz im Gegensatz zu unserer ursprünglichen Tumorzellinie BSp73AS, die überhaupt kein Metastasierungsverhalten zeigt.

**Der monoklonale Antikörper I.IASML hemmt Metastasenbildung in der Ratte**

In einer Serie von Versuchen zur Metastasenbildung der Tumorzellinie BSp73ASML in isogenen Ratten wurden Zellen subkutan injiziert und zu verschiedenen Zeiten der monoklonale Antikörper I.IASML intraperitoneal in Abständen von zwei bis drei Tagen vor und nach der Tumorgabe gespritzt. Im Rahmen dieses immunologischen Protokolls wurde auch bestimmt, wie die Immunantwort der Ratte gegenüber dem injizierten Antikörper stattgefunden hat. Es entstehen nämlich Antimaus-Immunglobulinantikörper, sowie auch Antiidiotyp-Antikörper. Das Ergebnis dieser Serie von Versuchen ist, daß das Anwachsen und die Metastasierung des Tumors durch Injektion von I.IASML stark verzögert wird. Diese Verzögerung läßt in ihrer Kinetik den Schluß zu, daß der Antikörper mit einem primären Prozeß der Metastasierung interferiert. Der Versuch zeigt uns, daß die vom Antikörper erkannte Proteinstruktur an der Oberfläche der metastasierenden Zellen eine Rolle in dem Metastasierungsprozeß hat und daß therapeutische und diagnostische Pläne realistisch sind.

**Isolierung der homologen menschlichen Sequenz zum EBZ kodierenden Sequenzteil der Ratten-cDNA.**

Für menschliche Tumorzellen in Kultur besteht natürlich nicht ohne weiteres die Möglichkeit, entsprechend den Rattensystemen, nachzuweisen, ob sie auch noch metastasierende Eigenschaften beibehalten. Experimente mit immundefizienten Mäusen ermöglichen nur senr eingeschränkt Aussagen über das Metastasenpotential beim Menschen. Deshalb mussten relativ viele Tumorzellinien, die zu lange zurückliegenden Zeitpunkten irgendwo auf der Welt in Kultur genommen worden sind, durchgetestet werden, ob sie die Sequenzen exprimieren, die wir für die Rattenmetastasen nachweisen konnten. Es ist gelungen, eine solche Tumorzellinie zu finden. Sie entstammt einem großzelligen Lungenkarzinom des Menschen und trägt die Nummer: LCLC97. In dieser Tumorzellinie lassen sich drei definierte RNA-Spezies nachweisen (Größen: 5,5; 3,4 und 2,8 kb), die sich ganz entsprechend den RNAs verhalten, die in den metastasierenden Tumorzellinien der Ratte nachweisbar sind. Sie hybridisieren nämlich sowohl mit der Probe A als auch mit den Proben B und C, d.h. daß auch diese menschlichen RNA Spezies über weite Bereiche zur cDNA pMeta-I identisch sind (85%).

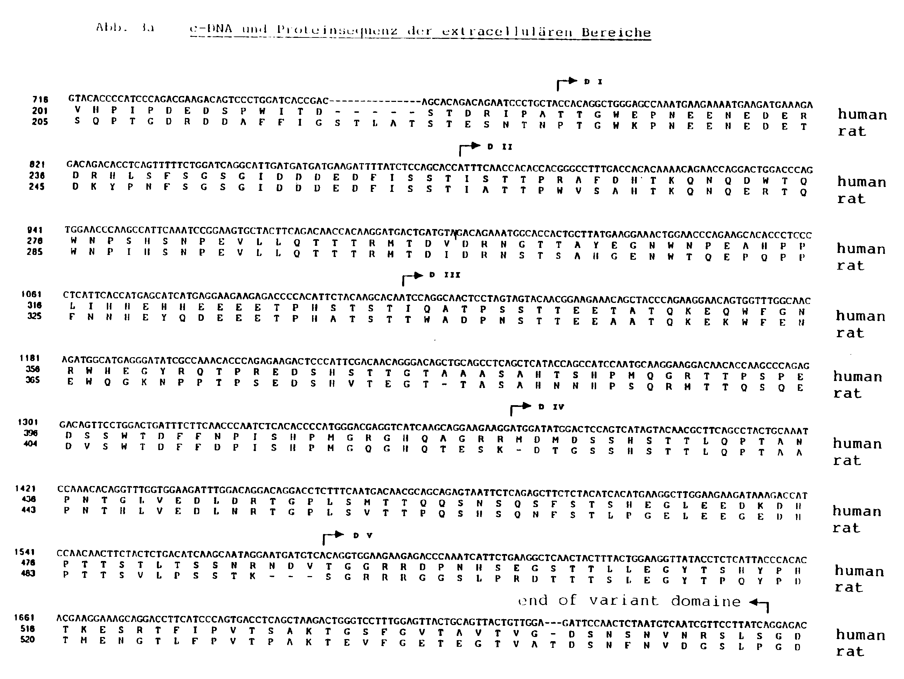

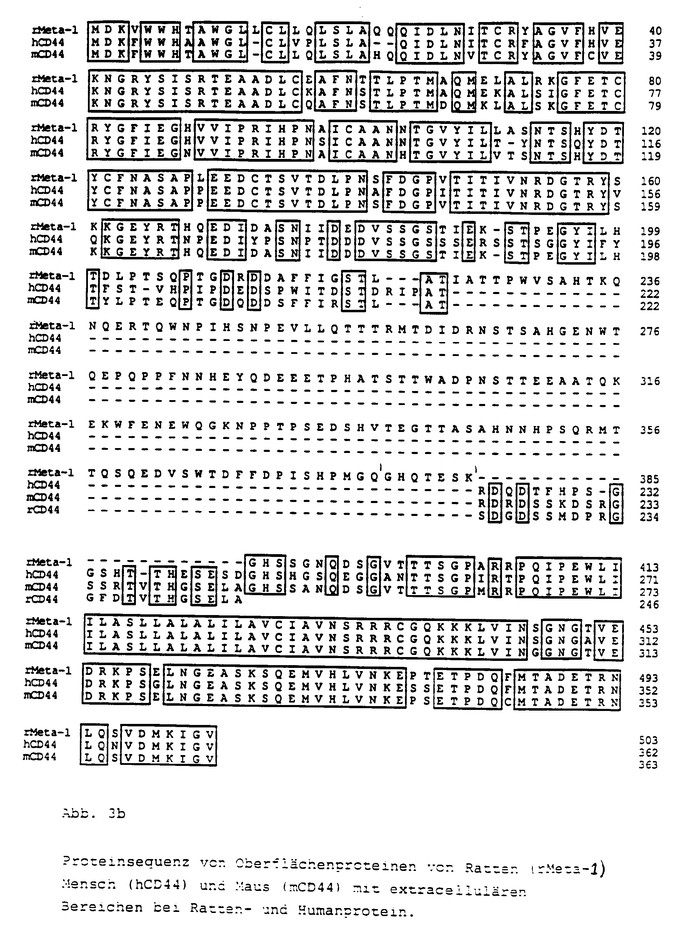

Der monoklonale AntiKörper I.IASML reagierte allerdings nicht mit dieser Tumorzelle, d.h. das von dem Antikörper erkannte Stück Protein muß sich im Bereich der Antigendeterminante von den Proteinen unterscheiden, die auf der Oberfläche der menschlichen Tumorzelle existieren. Für die Nichtreaktivität reichen schon geringste Abweichungen aufgrund der hohen Spezifität der Antikörper. Die menschliche Tumorzelle LCLC97 diente nun dazu, eine cDNA-Bibliothek zu konstruieren. Aufgrund der hohen Übereinstimmung zwischen den Ratten- und Menschsequenzen konnte ein cDNA Klon isoliert werden, der Homologie zur Probe A zeigte. Die menschlicne cDNA wurde sequenziert. In der Abbildung 3 (a,b) ist die Primärsequenz und die davon abgeleitete Aminosäuresequenz gezeigt. Man kann erkennen, daß über große Bereiche weitgehende Identität zwischen der Ratten- und der menschlichen Sequenz existiert. Diese menschliche Sequenz, sowie auch die davon abgeleitete Aminosäuresequenz ist ebenfalls Gegenstand dieser Patentschrift.

## Ausführungsbeispiele:

## Zellen und Antikörper:

Folgende klonierte Bsp Zellinien wurden für die Untersuchung eingesetzt: BSp73 14ASML-1 und 10AS-7 und in Kultur gehalten wie beschrieben bei Matzku et al., (1983); außerdem die bei Neri et al., (1982) beschriebenen Mammakarzinomzellinien Monoklonale Antikörper gegen BSp73 ASML Membranproteine wurden durch Immunisierung von Balbc Mäusen hergestellt. Nach Isolierung der Milzzellen einer immunisierten Maus wurden diese fusioniert mit Ag8 Myelomzellen zur Immortalisierung nach der Methode zur Herstellung monoklonaler Antikörper von Köhler (1981). Die dann erhaltenen Hybridomzellen wurden einem Screeningverfahren unterzogen, um solche herauszufinden, die spezifisch Antikörper gegen BSp73ASML produzieren, nicht aber gegen BSp73AS und normale Rattenfibroblasten-Zellen. Die genaue Vorgehensweise ist beschrieben, ebenso wie bei Matzku et al., (1989).

Monoklonale Antikörper (mAk) produzierende Hybridomzellen mit der entsprechenden Spezifität wurden expandiert in der Gewebekultur und die in das Medium abgegebenen mAk durch Ammoniumsulfatfällung und Säulenchromatographie (Protein A-Sepharose und MonoQ) stark angereichert und in dieser Form für die Untersuchungen eingesetzt. Einer davon ist mAkl.IASML.

## Immunfluoreszenz:

Zur Darstellung das varianten CD44 Moleküls auf verschiedenen Tumorzellen wurden diese in Kultur genommen, dann mit phosphatgepufferter Kochsalzlösung (PBS) gewaschen und mit I.IASML für 30 Minuten bei 4o C inkubiert. Als sekundärer Antikörper zum Nachweis der Bindung wurde ein Rhodamin-gekoppeltes Kaninchen Anti-Maus IgG eingesetzt und im Fluoreszenzmikroskop dargestellt.

## Konstruktion der cDNA Expressionsbibliotheken und Immunoscreening

PolyA+ RNA aus BSp73ASML Zellen wurde mit Oligo (dT) und Hexanukleotiden unterschiedlicher Zusammensetzung "geprimt" und mit reverser Transkriptase aus AMV der erste Strang der cDNA synthetisiert. Der zweite Strang der cDNA wurde mit E.coli DNA Polymerasel, RNaseH und E.coli Ligase hergestellt und anschließend die doppelsträngige cDNA mit T4DNA Polymerase an den Enden begradingt. Die Vektoren pEX1,2 und 3 (Stanley und Luzio, 1984), die die Fusionierung der cDNA in 3 verschiedenen Leserastern ermöglicht, wurden mit Smal Restriktionsendonuklease aufgeschnitten und mit der cDNA ligiert (T4 DNA Ligase). Kompentente E.coli DH5 (pCI857) Bakterien, die einen temperaturempfindlichen Repressor produzieren, werden mit den pEX-cDNA Konstrukten transfiziert und auf Nylonfiltern kultiviert. Das Gen für den temperaturempfindlichen Repressor RCI857 liegt auf dem Plasmid pCI857, das kompatibel ist mit den pEX Plasmiden. Bei 28°C ist der $IP_R$ Promoter, der die Synthese der Fusionsproteine steuert, abschaltet. Durch Temperaturerhöhung auf 42°C wird der CI Repressor inaktiviert und die Synthese von β-Galaktosidase/ASML Fusionsproteinen massiv in Gang gesetzt. Die hitze-induzierten Bakterienkolonien werden anschließend mit Chloroformdampf denaturiert auf den Filtern und diese dann in PBS inkubiert, das 3% Trockenmilchpulver, Lysozym und DNase enthält. Die an das Nylonfilter fixierten bakteriellen Fusionsproteine werden jetzt mit mAkI.IASML inkubiert und nach Auswaschen von unspezifisch gebundenem mAk, zum Nachweis der Bindung als sekundärer Antikörper 125J markierter Kaninchen Anti-Maus IgG eingesetzt. Nach Autoradiographie wurden positive Klone aus dem originalen Bakterienfilter isoliert und weitergehend analysiert. Ein Klon, der ein Fusionsprotein synthetisierte, das mit I.IASML spezifisch reagierte, war pEX34. Das in dem Bakterienklon enthaltene pEX Plasmid trägt 167 Nukleotide cDNA, die u.a. für das Epitop (bzw. die Antigendeterminante) kodieren, dessen Spezifität mAKI.IASML trägt.

Die Isolierung der Gesamtlängen cDNA mMeta-I erfolgte dann nach Standardmethoden.

## Immunisierung der Ratten mit mAkI.IASML

BDXRatten, die syngen sind zu den BSp73 Tumorzellen, wurde subkutan bzw. intraperitoneal mAkI.IASML (gekoppelt an Keyhole Limpet Haemocyanine) injiziert, zusammen mit komplettem Freunds Adjuvant. Die erste Injektion erfolgte 10, 7 und 3 Tage vor der Injektion der BSpASML Zellen (in das Fußfettpolster), die folgenden dann 3, 7, 11, 14 und 21 Tage danach. Nach 28 Tagen werden die Ratten getötet, die verschiedenen Lymphknoten präpariert und gewogen und makroskopisch sichtbare Lungenmetastasen gezählt.

## Zusammenhang zwischen der Expression von varianten CD44 Oberflächenproteinen und metastatischem Potential

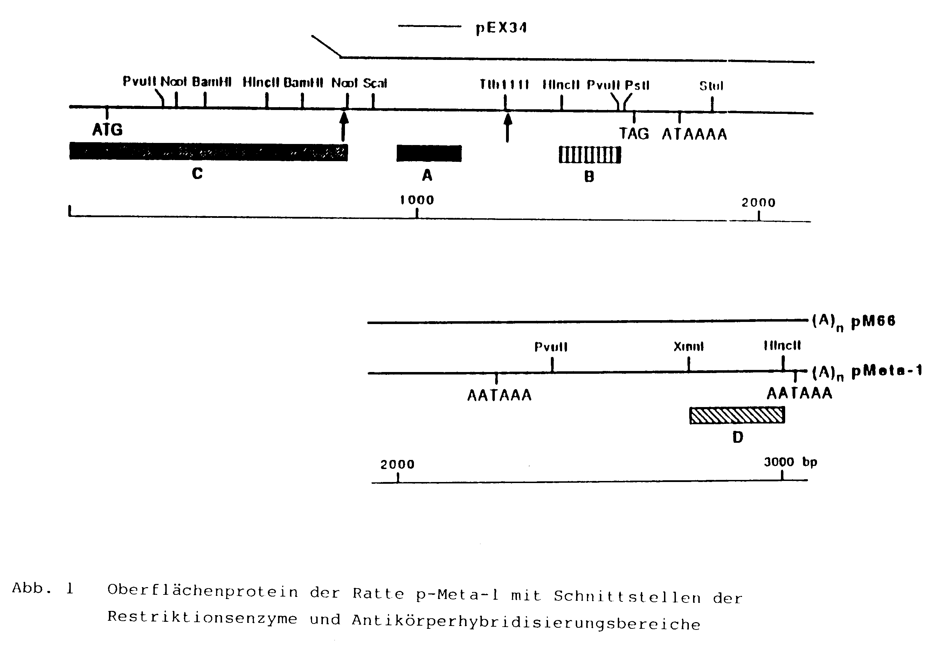

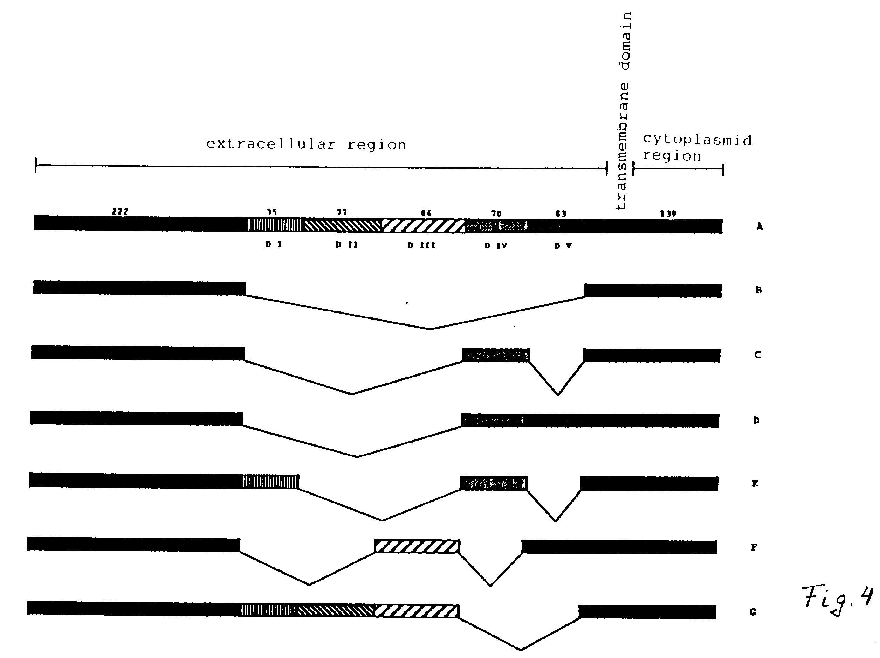

Um festzustellen, ob die Expression von varianten CD44 Glucoproteinen lediglich eine Eigenschaft der untersucnten BSp73ASML Zellinie ist, oder ob die Expression mit dem metastatischen Potential in Zusammenhang gebracht werden kann, wurde eine andere Serie von Rattentumorzellinien, die sich von dem 13762NF-Mammacarzinom ableitet (Neri et al, 1982) untersucht. Ferner wurden Zellinien, die sich vom Primär-Tumoren ableiten (MTPa, MTC, MTF7 und MTA (Gruppe 1)) mit Zellinien verglichen, die sich von Lymphknoten- und Lungenmetastasen ableiten (MTLy, MTLn2, MTLn3 (Gruppe 2)). Das Muster der von CD44 abgleiteten RNA ist in Figur 4 wiedergegeben, wobei Probe A, B und D den auf Seite 4 der Anmeldung sowie der Abbildung 1 beschriebenen Proben entsprechen. Zellen der Gruppe 1 zeigen alle ein normales CD44-Muster mit Probe B. Zellen der Gruppe 2 dagegen zeigen ein davon unterscniedliches Muster. Die RNA ist größer als die RNA der Gruppe 1 und entspricnt der RNA von BSp73ASML. Kleinere RNAs gehen verloren bei der Hybridisierung mit Probe D. Das übrige Muster zeigt die Ähnlichkeit zwischen den beiden Rattentumorsystemen.

Auch mit der Probe A entspricht das RNA-Muster der Gruppe 2 dem von BSp73ASML. Während Probe A mit RNA aus BSp73AS nicht hybridisiert, zeigt sich eine kleine diffuse RNA-Bande von ungefähr 2,5 kb bei Zellen der Gruppe 1. RNase und SI Nuklease Schutzanalyse zeigen ebenfalls die strukturelle Ähnlichkeit. Aus diesen Ergebnissen scheint ein Wechsel in dem Spaltungsmuster und der Expression von varianten CD44 RNAs mit der Bildung von Metastasen aufzutreten.

## Übertragung des metastatischen Potentials auf nicht metastasierende BSp73AS-Zellen durch Überexpression von p Meta-I.

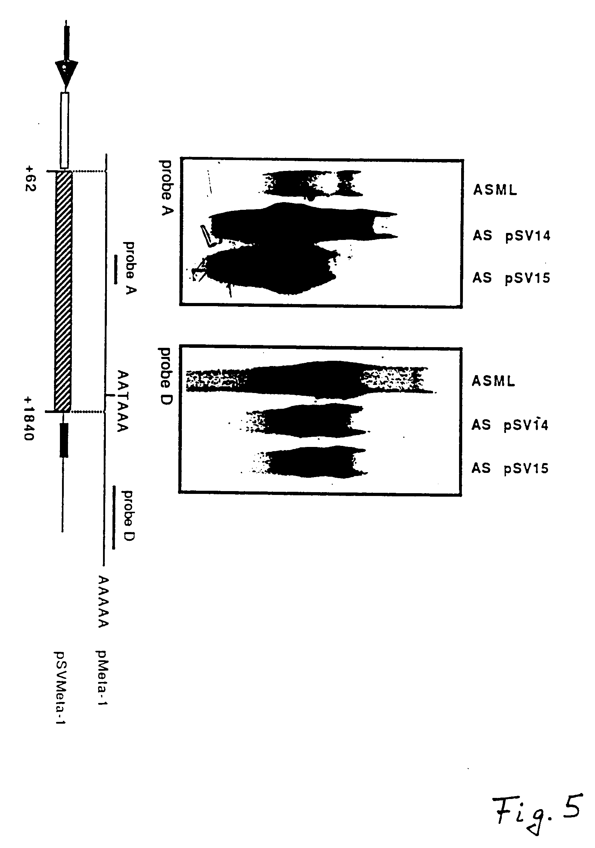

Der Zusammenhang der Expression von varianten CD44 Spezies mit dem metastatischen Potential in zwei Serien von Rattentumoren weist auf eine kausale Rolle dieser Glycoproteine im metastatischen Prozeß hin. Um diese zu untersuchen, wurde p-Meta-I in BSp73AS-Zellen übertragen und untersucht, ob sich dadurch das Verhalten der Zellen ändert. Die komplette kodierende Region des pMeta-I (Fig. 2) wurde unterhalb des SV40-Promotors eingefügt und dieses Gebilde (Diagramm in Figur 5) zusammen mit PSV2neo in BSp73AS-Zellen eingeführt. Einzelne G418-resistente und pMeta-I-exprimierende Kolonien wurden erhalten. Das RNA-Muster von 2 dieser Kolonien ist in Figur 5 wiedergegeben. Die Hyridisierung der varianten CD44 spezifischen Probe A zeigt ein dominantes Transkript von ungefähr 2.2 kb, welches in der Größe der kleinsten häufigen RNA, welche in BSp73ASML-Zellen transkribiert wird, entspricht (Figur 5). Die transfizierten Zellen enthalten jedoch ungefähr 10 mal soviel dieser RNA wie BSp73ASML.

Andere Größenordnungen werden in einer der transfizierten Zellen (BSp73AS-pSVMetal-14) beobachtet, was von dem Ort der Plasmid-Integration abhängig sein könnte. Ein pSV2neo Simulationsübertragungsklon (nicht wiedergegeben) und die BSp73AS-Empfängerzellen enthalten keine RNA, die komplementär zu Probe A ist. Um die endogenen normalen CD44-Transkriptionen (ohne die Extradomäne des pSVMeta-I) in den Transfekten zu entdecken, wurde das Filter gestript und mit Probe D rehybridisiert. Dieser Teil der nicht übersetzten 3' Sequenz ist in dem Expressionsklon nicht enthalten (vgl. Figur 5). Probe D weist zwei Haupttranskripte von 2,9 und 4,9 kb in der RNA der beiden Transfekte nach (Figur 5, rechte Spalte) sowohl in der Kontrolle BSp73AS als auch in der nicht abgebildeten BSp73ASpSVneo.

Ungefähre Quantifizierungen der verschiedenen übereinstimmenden Hybridisierungen zeigen, daß die Transfekte ungefähr 5 x soviel von den varianten CD44 RNAs exprimieren, welche durch das Expressionsplasmid transkribiert sind, wie die endogenen Gentranskripte.

Die überexprimierte cDNA wird in ein Protein übersetzt. Die beiden Transfekte, die in Figur 5 dargestellt sind, synthetisieren mAbl.IASML-immunfärbbare Proteine der gleichen scheinDaren Größe, nämlich ein Hauptprodukt von 150 kDa und eine schwächere Bande bei 100 kDa. Da die cDNA-Sequenz ein primäres Proteinprodukt von nur 503 Aminosäuren kodiert (entspricht ca. 60.000 Dalton), müssen alle sichtbaren Banden modifizierte Formen darstellen. Die 150 kDa-Bande läuft zusammen mit einer der modifizierten Formen von varianter CD44, welche in der metastasierenden Zelle BSp73ASML exprimiert wird. BSp73AS oder simulationsübertragene BSp73ASpSVneo Zellen besitzen dieses Protein nicht. Wie in BSp73ASML-Zellen ist das Epitop der von den Transfekte exprimierten Zellen an der Zelloberfläche freiliegend.

Um zu beweisen, daß die Expression von varianter CD44 ausreicht, um BSp73AS-Zellen ein metastatisches Potential zu verleihen, wurden Transfekte in syngene BDX-Ratten (spontanes Metastase-Protokoll) injiziert. In früheren Experimenten breiteten sich vom Ort der Injektion metastatische Tumorzellen BSp73ASML schnell aus und waren ungefähr 10 Tage nach der Injektion vollständig verteilt (Matzku, 1984). Alle lokalen Tumore wurden daher durch Amputationen am 10. Tag entfernt. Alle Träger von BSp73ASML-Zellen und alle Tiere, die mit einem der überexprimierenden Transfekte injiziert waren, entwickelten Lungenmetastasen (Tabelle 1). Der Verlauf der Metastasenbildung war vergleichbar schnell, innerhalb von 5 - 8 Wochen nach der Injektion. Tiere, die BSp73AS-Zellen oder Simulationstransfekte erhalten hatten, waren nach dieser Zeit vollständig gesund (außer durch die Amputation) und auch nach 5 Monaten konnten keine Metastasen festgestellt werden.

Trotz einer überraschenden Ähnlichkeit in der starken Metastasebildung gibt es einige interessante Unterschiede.

BSp73ASML-Zellen erreichen in allen Tieren die Lymphknoten und führen zu einer massiven Vergrößerung verschiedener Knoten im Bereich der Leiste inguinaler und neben der Aorta (Tabelle 1). Ein Transfekt (BSp73AS-pSVMetal-14) verursacht Lymphknotenvergrößerungen in 3 von 8 Tieren, obwohl alle Tiere multiple Lungenmetastasen entwickeln (Tabelle 1). Mit dem anderen Transfekt (BSp73AS-pSVMetal-15) ist keine Lymphknotenvergrößerung feststellbar. Die Transfekte scheinen daher in der Lage zu sein, in der Lunge Kolonien zu bilden ohne eine obligatorische Wachstumsphase in den Lymphknoten.

Das Experiment gemäß Tabelle 1 deutet ferner auf einen anderen Unterschied zwischen BSp73AS-Transfekten und BSp73ASML hin. Die individuellen Lungenmetastasen sind makroskopisch sichtbar, während die von von BSp73ASML klein und zahlreich sind, jedoch entwickeln in einer größeren Serie mit BSp73ASML (Reber et al., 1990) 11 von 20 Tieren 5 - 20 größere Knoten pro Lunge, als die Transfekte.

Um festzustellen, daß die gebildeten Metastasen von den injizierten Transfekte hervorgerufen wurden, und um die unwahrscheinliche Möglichkeit einer spontanen Mutation, welche ein metastatisches Potential überträgt, auszuschließen, wurden die epitop-positiven Proteine in den Gesamtlungenextrakten und in Extrakten von rekultivierten metastaseerzeugenden Zellen bestimmt. Im gesamten Lungenextrakt sowie in den Extrakten eines spezifischen Lungenknotens von einem Tier, das BSpAS-pSVMetal-15-Transfekte erhalten hat, ist das 150 kDa Glycoprotein nachweisbar. Der G418-resistente Stamm exprimiert beim in vitro-Wachstum ein Protein desselben scheinbaren Molekular gewichtes.

## Diagnostik und Therapie

1. Analyse menschlichen Tumormaterials durch in situ Hybridisierungen mit den vorhandenen menschlichen pMeta-I Sequenzen. Diese Versuche sind als Vorversuche gedacht, bevor ein Ak zur Verfügung stent, der den menschlichen EZB erkennt.

2. Herstellung von Antikörpern gegen den menschlichen EZB. Klonierung der menschlichen pMetal Sequenzen in bakterielle Expressionsvektoren, so daß Fusionen mit β-Galaktosidase oder Tryptophan E-Produkt entstehen. Immunisierung von Kaninchen mit diesen Fusionsproteinen, bzw. mit synthetisierten Peptiden aus dem EZB (gekoppelt an Trägermoleküle). Isolierung der polyvalenten bzw. monospezifischen Antikörper.

**Einsatzmöglichkeiten:**

- Immunhistologische Untersuchungen von klinischem Tumormaterial (Diagnostik).
- Nachweis von löslichem EZB im Serum von Patienten mit Hilfe von ELISA-Tests (Diagnostik).
- Konstruktion von Toxin-gekoppelten Antikörpern, um mit Hilfe des Antikörpers das Toxin in den Tumor/Metastasenbereich zu bringen (Therapie).
- Konstruktion von Antikörpern mit zwei definierten Antigenbindungsstellen. Durch diese Doppelspezifität soll versucht werden, cytotoxische Reaktionen im Metastasengebiet auszulösen (z.B. Anti CD2- oder CD3-Kopplung) (Therapie),

3. Produktion von hMeta-I Protein durch Transfektion von menschlichen oder Rattenzellen mit einem Expressionsvektor, der die vollständige hMeta-I cDNA Sequenz trägt; bzw. Reinigung aus LCLC97-Zellen.

**Einsatzmöglichkeiten:**
- Injektion des Proteins oder Teile desselben, um die Gewebe-Bindungsstellen der Tumorzellen zu blockieren.
- Nach Charakterisierung der Bindungsstellen wäre auch ein Einsatz zur Therapie denkbar, der darin beruhen könnte, große Mengen von Bindungsprotein zu injizieren, das dann die wandernden Tumorzellen blockieren würde.

**Literaturliste:**

Goldstein, L. A., Zhou, D. F. H., Picker, L. J., Minty, C. N., Bargatze, R. F., Ding, J. F. and Butcher, E. C. (1989) A human lymphocyte homing receptor, the hermes antigen, is related to cartilage proteoglycan core and link proteins. Cell 56: 1063-1072.

Hart, I. R., Goode, N. T. and Wilson, R. E. (1989) Molecular aspects of the metastatic cascade. Biochim. Biophys. Acta 989: 65-84.

Idzerda, R. L., Carter, W. G., Nottenburg, C., Wayner, E. A., Gallatin, W. M. and St. John, T. (1989) Isolation and DNA sequence of a cDNA clone encoding a lymphocyte adhesion receptor for high endothelium. Proc. Natl. Acad. Sci. U.S.A. 86: 4659-4663.

Köhler, G. (1981) In: I. Lefkovits and B. Pernis (eds). Immunological Methods. Vol. 2 p. 285. N.Y. Academic Press.

Matzku, S., Komitowski, Mildenberger and Zöller, M. (1983) Characterization of Bsp 73, a spontaneous rat tumor and its in vivo selected variants showing different metastasizing capacities. Inv. Met. 3: 109-123.

Matzku, S., Wenzel, A., Liu, S. and Zöller, M. (1989). Antigenic differences between metastatic and nonmetastatic BSp73 rat tumor variants characterized by monoclonal antibodies. Cancer Res. 49: 1294-1299.

Neri, A., Welch, D., Kawaguchi, T. and Nicolson, G. L. (1982) Development and biologic properties of malignant cell sublines and clones of spontaneously metastasizing rat mammary adenocarcinoma. J. Natl. Cancer Inst. 68: 507-517.

Nicolson, G. L. (1987) Tumor cell instability, diversification, and progression to the metastatic phenotype: from oncogene to oncofetal expression. Cancer Res. 47: 1473-1487.

Nottenburg, C., Rees, G. and St. John, T. (1989) Isolation of mouse CD44 cDNA: structural features are distinct from the primate cDNA. Proc. Natl. Acad. Sci. U.S.A. 86: 8521-8525.

Stamenkovic, I., Amiot, M., Pesando, J. M. and Seed, B. (1989) A lymphocyte molecule implicated in lymph node homing is a member of the cartilage link protein family. Cell 56: 1057-1062.

Stanley K.K. and Luzio, J.P. (1984), Construction of a new family of high efficiency bacterial expression vectors: identification of cDNA clones coding for human liver proteins. EMBO J. 3: 1429-1434.

Wenzel, A. (1986) Charakterisierung von Differenzierungsantigenen auf dem Rattentumor Bsp 73 mit Hilfe monoklonaler Antikörper. Diplomarbeit, Universität Karlsruhe

Zhou, D. F. H., Ding, J. F., Picker, L. F., Bargatze, R. F., Butcher, E. C. and Goeddel, D. V. (1989) Molecular cloning and expression of Pgp-I - The mouse homolog of the human H-CAM (Hermes) lymphocyte homing receptor. J. Immunol. 143: 3390-3395.

Tabelle 1

Metastatische Ausbreitung von BSp73AS-Zellen, welche variante CD44 cDNA pMeta-1 exprimieren***

| Tumor Clone | Lokales Auftreten | Verteilung bei Metastatischer Autopsie *LN ing | *LN par | Lunge |
|---|---|---|---|---|
| BSp73ASML | 0/8 | 8/8<br>ø 1.5-2.5** | 8/8<br>ø 2.5-5.0 | 8/8<br>miliary |
| BSp73AS-pSVMeta1-14 | 0/8 | 3/8<br>ø 0.3-1.2 | 3/8<br>ø 1.0-4.5 | 8/8<br>multiple, ø 0.3-5.0 |
| BSp73AS-pSVMeta1-15 | 0/8 | 0/8 | 0/8 | 8/8<br>5-20, ø 0.3-10.0 |
| BSp73AS | 1/8 | 0/8 | 0/8 | 0/8 |
| BSp73AS-pSVneo | 0/8 | 0/8 | 0/8 | 0/8 |

** mittlerer Durchmesser in mm

*** Die Tabelle gibt das Stadium 60 Tage nach Injektion der angegebenen Zellen wieder.

*LN = Lymphknoten

## Patentansprüche

1. DNA-Fragment, welches für einen Teil eines Oberflächenproteins metastasierender Tumorzellen kodiert, wobei dieses DNA-Fragment ausgewählt ist aus
   a) den Nukleotid-Sequenzen
      ...rMeta-I...

ATT GCA ACT ACT CCA TGG GTT TCT GCC CAC ACA AAA CAG AAC
CAG GAA CGG ACC CAG TGG AAC CCG ATC CAT TCA AAC CCA GAA
GTA CTA CTT CAG ACA ACC ACC AGG ATG ACT GAT ATA GAC AGA
AAC AGC ACC AGT GCT CAT GGA GAA AAC TGG ACC CAG GAA CCA
CAG CCT CCT TTC AAT AAC CAT GAG TAT CAG GAT GAA GAG GAG
ACC CCA CAT GCT ACA AGC ACA ACC TGG GCA GAT CCT AAT AGC
ACA ACA GAA GAA GCA GCT ACC CAG AAG GAG AAG TGG TTT GAG
AAT GAA TGG CAG GGG AAG AAC CCA CCC ACC CCA AGT GAA GAC
TCC CAT GTG ACA GAA GGG ACA ACT - GCC TCA GCC CAC AAC
AAC CAT CCA AGT CAA AGA AGT ACA ACA CAG AGT CAA GAG GAT
GTT TCA TGG ACC GAT TTC TTC GAC CCA ATC TCA CAT CCA ATG
GGA CAA

und

hMeta-I...

AAC CCA AGC CAT TCA AAT CCG GAA GTG CTA CTT CAG ACA ACC
ACA AGG ATG CAT GAT GTA GAC AGA AAT GGC ACC ACT GCT TAT
GAA GGA AAC TGG AAC CCA GAA GCA CAC CCT CCC CTC ATT CAC
CAT GAG CAT CAT GAG GAA GAA GAG ACC CCA CAT TCT ACA AGC
ACA ATC CAG GCA ACT CCT AGT AGT ACA ACG GAA GAA ACA GCT
ACC CAG AAG GAA CAG TGG TTT GGC AAC AGA TGG CAT GAG GGA
TAT CGC CAA ACA CCC AGA GAA GAC TCC CAT TCG ACA ACA GGG
ACA GCT GCA GCC TCA GCT CAT ACC AGC CAT CCA ATG CA

b) Nukleotid-Sequenzen, welche gegenüber den unter a) genannten DNA-Sequenzen degeneriert sind und/oder allele Variationen darstellen.

2. DNA-Fragmente gemäß Anspruch 1, dadurch gekennzeichnet, daß sie Nukleotid-Sequenzen enthalten, die mit einer der in Anspruch 1 a) genannten Nukleotid-Sequenz hybridisieren und für ein vollständiges Oberflächenglykoprotein metastasierender Tumorzellen kodieren.

3. DNA-Fragment nach Anspruch 2, dadurch gekennzeichnet, daß es mit einer der in Anspruch 1 genannten Nukleotidsequenz hybridisiert, welche mindestens 85 % Homologie zum Reaktionspartner aufweist.

4. Rekombiniertes DNA-Molekül, bestehend aus einer vektoriellen Nukleotidsequenz und einem DNA-Fragment nach Ansprüchen 1, 2, oder 3.

5. Rekombinantes DNA-Molekül nach Anspruch 4, dadurch gekennzeichnet, daß es ein Expressionsvektor ist.

6. Transformierte Wirtszelle, welche ein DNA-Fragment nach Ansprüchen 1, 2 oder 3, oder ein rekombiniertes DNA-Molekül nach Ansprüchen 4 oder 5 enthält.

7. Polypeptid, welches durch ein DNA-Fragment nach Anspruch 1 kodiert wird.

8. Polypeptid, herstellbar durch ein rekombinantes DNA-Molekül nach Ansprüchen 4 oder 5, welches ein DNA-Fragment gemäß Anspruch 1 umfaßt.

9. Polypeptid nach Ansprüchen 7 und 8, umfassend die Aminosäuresequenzen
...r-Protein...

I A T T P W V S A H T K Q N Q E R T Q W N P I H S N P E V L
L Q T T T R M T D I D R N S T S A H G E N W T Q E P Q P P F
N N H E Y Q D E E E T P H A T S T T W A D P N S T T E E A A
T Q K E K W F E N E W Q G K N P P T P S E D S H V T E G T T
A S A H N N H P S Q R M T T Q S Q E D V S W T D F F D P I S
H P M G Q G H Q T E S K

und h-Protein

I S S T I S T T P R A F D H T K Q N Q D W T Q W N P S H S N
P E V L L Q T T T R M T D V D R N G T T A Y E G N W N P E A
H P P L I H H E H H E E E E T P H S T S T I Q A T P S S T T
E E T A T Q K E Q W F G N R W H E G Y R Q T P R E D S H S T
T G T A A A S A H T S H P M Q G R T T P S P E D S S W T D F
F N P I S H P M G R G H Q A G R R

und deren allele Variationen und Glykosylierungsprodukte.

10. Poly- und monovalente Antikörper, die mit einem Epitop eines Polypeptids nach Ansprüchen 7 bis 9 reagieren.

11. Verwendung eines DNA-Fragments nach Anspruch 1, deren komplementäre Stränge, Derivate und Teile davon zur Identifizierung, Herstellung oder Isolierung einer Nukleotidsequenz oder Teilen davon, die für einen Teil eines Oberflächenglykoproteins metastasierender Tumorzellen kodieren.

12. Verwendung eines Antikörpers nach Anspruch 10 zur Identifizierung, Herstellung oder Isolierung eines Polypeptids nach Ansprüchen 7 bis 9.

13. Mittel zur Diagnose von metastasierenden Tumoren und/oder Metastasen, welches mindestens einen Antikörper nach Anspruch 10 enthält.

14. Mittel nach Anspruch 13, dadurch gekennzeichnet, daß der Antikörper mit einem Enzym gekoppelt, mit einem Farbstoff und/oder mit einem Radioisotop markiert ist.

15. Antikörper gegen Proteine gemäß Anspruch 7, hergestellt unter Verwendung von DNA gemäß Anspruch 1 oder Proteinen gemäß Anspruch 7.

16. Verwendung von Antikörpern und Proteinen oder Teilen davon gemäß Ansprüchen 15 und 7 zur Herstellung von Arzneimitteln, welche in der Tumor-Therapie verwendet werden.

17. Verwendung von Antikörpern gemäß Anspruch 15, welche mit cytotoxischen Agenzien gekoppelt sind zur Herstellung von Arzneimitteln für die Tumor-Therapie.

## Claims

1. DNA-fragment which codes for a part of a surface protein of metastasing tumour cells, whereby this DNA-fragment is selected from
a) the nucleotide sequences
...rMeta-I...

ATT GCA ACT ACT CCA TGG GTT TCT GCC CAC ACA AAA CAG AAC
CAG GAA CGG ACC CAG TGG AAC CCG ATC CAT TCA AAC CCA GAA
GTA CTA CTT CAG ACA ACC ACC AGG ATG ACT GAT ATA GAC AGA
AAC AGC ACC AGT GCT CAT GGA GAA AAC TGG ACC CAG GAA CCA
CAG CCT CCT TTC AAT AAC CAT GAG TAT CAG GAT GAA GAG GAG
ACC CCA CAT GCT ACA AGC ACA ACC TGG GCA GAT CCT AAT AGC
ACA ACA GAA GAA GCA GCT ACC CAG AAG GAG AAG TGG TTT GAG
AAT GAA TGG CAG GGG AAG AAC CCA CCC ACC CCA AGT GAA GAC
TCC CAT GTG ACA GAA GGG ACA ACT - GCC TCA GCC CAC AAC
AAC CAT CCA AGT CAA AGA AGT ACA ACA CAG AGT CAA GAG GAT
GTT TCA TGG ACC GAT TTC TTC GAC CCA ATC TCA CAT CCA ATG
GGA CAA

and

hMeta-1...

AAC CCA AGC CAT TCA AAT CCG GAA GTG CTA CTT CAG ACA ACC
ACA AGG ATG CAT GAT GTA GAC AGA AAT GGC ACC ACT GCT TAT
GAA GGA AAC TGG AAC CCA GAA GCA CAC CCT CCC CTC ATT CAC
CAT GAG CAT CAT GAG GAA GAA GAG ACC CCA CAT TCT ACA AGC
ACA ATC CAG GCA ACT CCT AGT AGT ACA ACG GAA GAA ACA GCT
ACC CAG AAG GAA CAG TGG TTT GGC AAC AGA TGG CAT GAG GGA
TAT CGC CAA ACA CCC AGA GAA GAC TCC CAT TCG ACA ACA GGG
ACA GCT GCA GCC TCA GCT CAT ACC AGC CAT CCA ATG CA

b) nucleotide sequences which are degenerated with regard to the DNA sequences mentioned under a) and/or represent allele variations.

2. DNA fragments according to claim 1, characterised in that they contain nucleotide sequences which hybridise with one of the nucleotide sequences mentioned in claim 1 a) and code for a complete surface glycoprotein of metastasing tumour cells.

3. DNA fragment according to claim 2, characterised in that it hybridises with one of the nucleotide sequences mentioned in claim 1 which has at least 85% homology with the reaction partner.

4. Recombined DNA molecule consisting of a vectorial nucleotide sequence and a DNA fragment according to claims 1, 2 or 3.

5. Recombinant DNA molecule according to claim 4, characterised in that it is an expression vector.

6. Transformed host cell which contains a DNA fragment according to claims 1, 2 or 3 or a recombined DNA molecule according to claims 4 or 5.

7. Polypeptide which is coded by a DNA fragment according to claim 1.

8. Polypeptide producable by a recombinant DNA molecule according to claims 4 or 5 which includes a DNA fragment according to claim 1.

9. Polypeptide according to claims 7 and 8, including the amino acid sequences

...r-protein...

I A T T P W V S A H T K Q N Q E R T Q W N P I H S N P E V L
L Q T T T R M T D I D R N S T S A H G E N W T Q E P Q P P F
N N H E Y Q D E E E T P H A T S T T W A D P N S T T E E A A
T Q K E K W F E N E W Q G K N P P T P S E D S H V T E G T T
A S A H N N H P S Q R M T T Q S Q E D V S W T D F F D P I S
H P M G Q G H Q T E S K

and h-protein

I S S T I S T T P R A F D H T K Q N Q D W T Q W N P S H S N
P E V L L Q T T T R M T D V D R N G T T A Y E G N W N P E A
H P P L I H H E H H E E E E T P H S T S T I Q A T P S S T T
E E T A T Q K E Q W F G N R W H E G Y R Q T P R E D S H S T
T G T A A A S A H T S H P M Q G R T T P S P E D S S W T D F
F N P I S H P M G R G H Q A G R R

and their allele variations and glycosylation products.

10. Poly- and monovalent antibodies which react with an epitope of a polypeptide according to claims 7 to 9.

11. Use of a DNA fragment according to claim 1, its complementary strands,derivatives and parts thereof for the identification, production or isolation of a nucleotide sequence or parts thereof which code for a part of a surface glycoprotein of metastasing tumour cells.

12. Use of an antibody according to claim 10 for the identification, production or isolation of a polypeptide according to claims 7 to 9.

13. Agent for the diagnosis of metastasing tumours and/or metastases which contain at least one antibody according to claim 10.

14. Agent according to claim 12, characterised in that the antibody is coupled with an enzyme, labelled with a coloured material and/or with a radioisotope.

15. Antibody against proteins according to claim 7 produced with the use of DNA according to claim 1 or proteins according to claim 7.

16. Use of antibodies and proteins or parts thereof according to claims 15 and 7 for the production of medicaments which are used in tumour therapy.

17. Use of antibodies according to claim 15 which are coupled with cytotoxic agents for the production of medicaments for tumour therapy.

**Revendications**

1. Fragment d'ADN, qui code pour une partie d'une protéine superficielle de cellules tumorales génératrices de métastases, ce fragment d'ADN étant choisi parmi
   a) les séquences nucléotidiques
      ...rMeta-1...

ATT GCA ACT ACT CCA TGG GTT TCT GCC CAC ACA AAA CAG AAC
CAG GAA CGG ACC CAG TGG AAC CCG ATC CAT TCA AAC CCA GAA
GTA CTA CTT CAG ACA ACC ACC AGG ATG ACT GAT ATA GAC AGA
AAC AGC ACC AGT GCT CAT GGA GAA AAC TGG ACC CAG GAA CCA
CAG CCT CCT TTC AAT AAC CAT GAG TAT CAG GAT GAA GAG GAG
ACC CCA CAT GCT ACA AGC ACA ACC TGG GCA GAT CCT AAT AGC
ACA ACA GAA GAA GCA GCT ACC CAG AAG GAG AAG TGG TTT GAG
AAT GAA TGG CAG GGG AAG AAC CCA CCC ACC CCA AGT GAA GAC
TCC CAT GTG ACA GAA GGG ACA ACT - GCC TCA GCC CAC AAC
AAC CAT CCA AGT CAA AGA AGT ACA ACA CAG AGT CAA GAG GAT
GTT TCA TGG ACC GAT TTC TTC GAC CCA ATC TCA CAT CCA ATG
GGA CAA

et

hMeta-I...

AAC CCA AGC CAT TCA AAT CCG GAA GTG CTA CTT CAG ACA ACC
ACA AGG ATG CAT GAT GTA GAC AGA AAT GGC ACC ACT GCT TAT
GAA GGA AAC TGG AAC CCA GAA GCA CAC CCT CCC CTC ATT CAC
CAT GAG CAT CAT GAG GAA GAA GAG ACC CCA CAT TCT ACA AGC
ACA ATC CAG GCA ACT CCT AGT AGT ACA ACG GAA GAA ACA GCT
ACC CAG AAG GAA CAG TGG TTT GGC AAC AGA TGG CAT GAG GGA
TAT CGC CAA ACA CCC AGA GAA GAC TCC CAT TCG ACA ACA GGG
ACA GCT GCA GCC TCA GCT CAT ACC AGC CAT CCA ATG CA

b) des séquences nucléotidiques, qui sont dégénérées par rapport aux séquences d'ADN citées sous a) et/ou représentent des variantes allèles.

2. Fragments d'ADN suivant la revendication 1, caractérisés en ce qu'ils comportent des séquences nucléotidiques qui s'hybrident avec une des séquences nucléotidiques citées dans la revendication 1 a) et qui codent pour une glycoprotéine superficielle complète de cellules tumorales génératrices de métastases.

3. Fragment d'ADN suivant la revendication 2, caractérisé en ce qu'il s'hybride avec une des séquences nucléotidiques citées dans la revendication 1 qui présente au moins 85 % d'homologie pour le partenaire réactionnel.

4. Molécule d'ADN recombiné, constituée d'une séquence nucléotidique vectorielle et d'un fragment d'ADN suivant l'une des revendications 1, 2 et 3.

5. Molécule d'ADN recombinant suivant la revendication 4, caractérisée en ce qu'elle est un vecteur d'expression.

6. Cellule hôte transformée, qui contient un fragment d'ADN suivant l'une des revendications 1, 2 et 3, ou une molécule d'ADN recombiné selon l'une des revendications 4 et 5.

7. Polypeptide qui est codé par un fragment d'ADN suivant la revendication 1.

8. Polypeptide, préparable par une molécule d'ADN recombinant suivant l'une des revendications 4 et 5, qui comporte un fragment d'ADN suivant la revendication 1.

**9.** Polypeptide suivant l'une des revendications 7 et 8, comportant les séquences d'aminoacides
...r-protéine...

I A T T P W V S A H T K Q N Q E R T Q W N P I H S N P E V L
L Q T T T R M T D I D R N S T S A H G E N W T Q E P Q P P F
N N H E Y Q D E E E T P H A T S T T W A D P N S T T E E A A
T Q K E K W F E N E W Q G K N P P T P S E D S H V T E G T T
A S A H N N H P S Q R M T T Q S Q E D V S W T D F F D P I S
H P M G Q G H Q T E S K

et h-protéine

I S S T I S T T P R A F D H T K Q N Q D W T Q W N P S H S N
P E V L L Q T T T R M T D V D R N G T T A Y E G N W N P E A
H P P L I H H E H H E E E E T P H S T S T I Q A T P S S T T
E E T A T Q K E Q W F G N R W H E G Y R Q T P R E D S H S T
T G T A A A S A H T S H P M Q G R T T P S P E D S S W T D F
F N P I S H P M G R G H Q A G R R

et leurs variantes allèles et produits de glycosylation.

**10.** Anticorps polyvalents et monovalents qui réagissent avec un épitope d'un polypeptide suivant l'une des revendications 7 à 9.

**11.** Utilisation d'un fragment d'ADN suivant la revendication 1, de ses brins complémentaires, de dérivés et de parties de celui-ci, pour l'identification, la préparation ou l'isolement d'une séquence nucléotidique ou de parties de celle-ci qui codent pour une partie d'une glycoprotéine superficielle de cellules tumorales génératrices de métastases.

**12.** Utilisation d'un anticorps suivant la revendication 10 pour l'identification, la préparation ou l'isolement d'un polypeptide suivant l'une des revendication 7 à 9.

**13.** Produit de diagnostic de tumeurs génératrices de métastases et/ou de métastases, qui comporte au moins un anticorps suivant la revendication 10.

**14.** Produit suivant la revendication 13, caractérisé en ce que l'anticorps couplé à une enzyme est marqué par un colorant et/ou un radio-isotope.

**15.** Anticorps contre des protéines suivant la revendication 7, préparés par l'utilisation d'ADN suivant la revendication 1 ou de protéines suivant la revendication 7.

**16.** Utilisation d'anticorps et de protéines ou de parties de celles-ci suivant les revendications 15 et 7, pour la préparation de médicaments qui sont utilisés dans la thérapie des tumeurs.

**17.** Utilisation des anticorps suivant la revendication 15, qui sont couplés à des agents cytotoxiques, pour la fabrication de médicaments destinés à la thérapie des tumeurs.

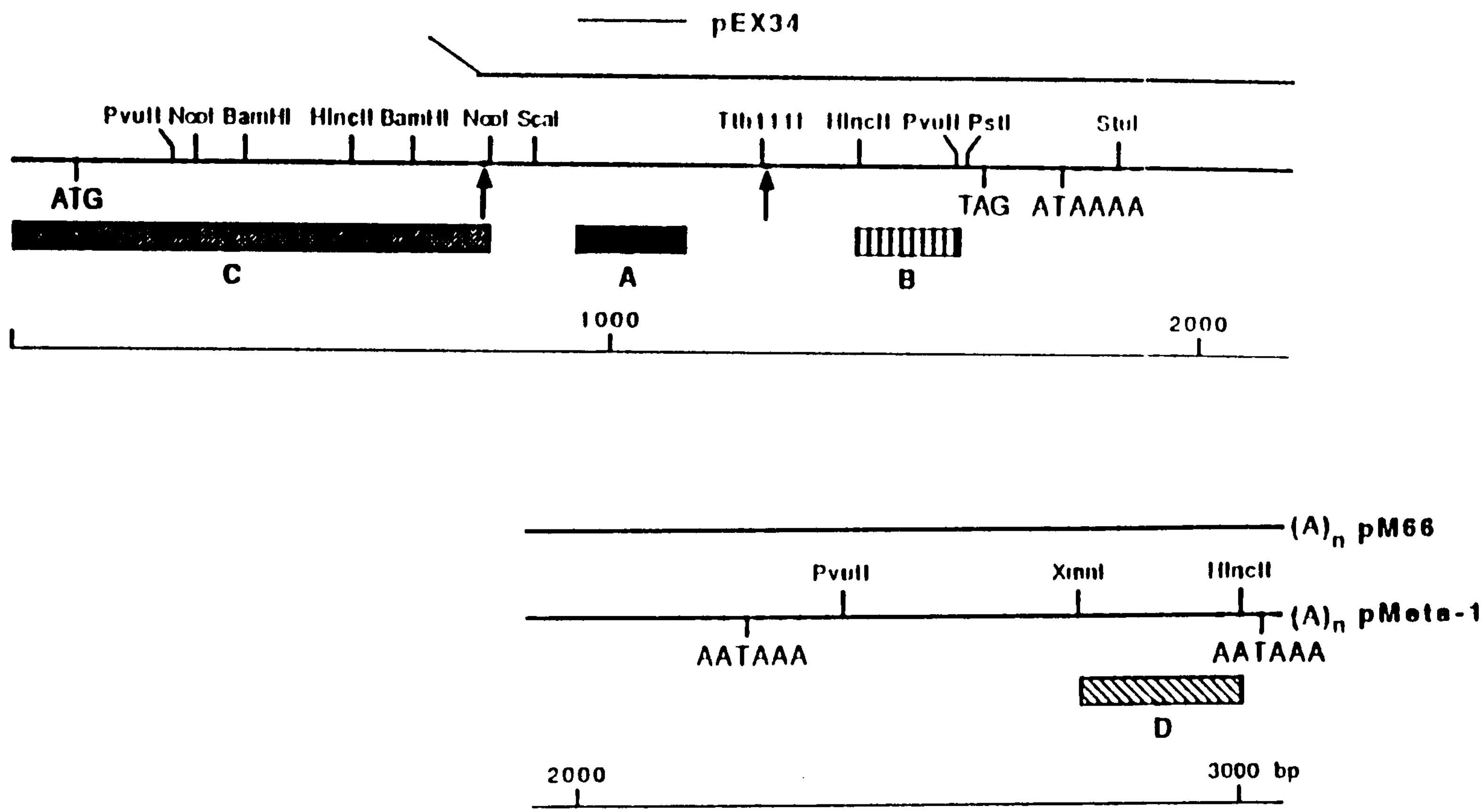


Abb. 1 Oberflächenprotein der Ratte p-Meta-1 mit Schnittstellen der Restriktionsenzyme und Antikörperhybridisierungsbereiche

```
1       CTCATTGCCCAGCAGCCCCCAGCCAGTGACAGGTTCCATTCACCCTCTTTGCCCCTTCCCCCGCGACCCTTTTCCAGAGGCTACTAGATCCTTTGGTTTCATCCTGCACATC

113     ATGGACAAGGTTTGGTGGCACACAGCTTGGGGACTACTTTGCCTCTTACAGTTGAGCCTGGCACAGCAGCAGATCGATTTGAATATAACCTGCCGTTACGCAGGTGTATTCCATGTGGAG
         M  D  K  V  W  W  H  T  A  W  G  L  L  C  L  L  Q  L  S  L  A  Q  Q  Q  I  D  L  N  I  T  C  R  Y  A  G  V  F  H  V  E

233     AAAAATGGCCGCTACAGTATCTCCAGGACTGAAGCAGCTGACCTCTGCGAGGCTTTCAACACCACCTTGCCCACCATGGCTCAGATGGAGTTAGCCCTGAGAAAGGGTTTGAAACATGC
         K  N  G  R  Y  S  I  S  R  T  E  A  A  D  L  C  E  A  F  N  T  T  L  P  T  M  A  Q  M  E  L  A  L  R  K  G  F  E  T  C

353     AGGTATGGGTTCATAGAAGGACACGTGGTAATCCCGAGGATCCACCCCAACGCTATCTGTGCAGCCAACAACACAGGAGTGTATATCCTCCTCGCATCCAACACCTCCCACTATGACACA
         R  Y  G  F  I  E  G  H  V  V  I  P  R  I  H  P  N  A  I  C  A  A  N  N  T  G  V  Y  I  L  L  A  S  N  T  S  H  Y  D  T

473     TATTGCTTCAATGCCTCAGCTCCTCTTGAAGAAGACTGTACATCAGTCACAGACCTACCCAATTCCTTCGATGGACCAGTTACCATAACTATTGTCAACCGTGATGGCACCCGCTACAGC
         Y  C  F  N  A  S  A  P  L  E  E  D  C  T  S  V  T  D  L  P  N  S  F  D  G  P  V  T  I  T  I  V  N  R  D  G  T  R  Y  S

593     AAGAAGGGCGAGTATAGAACACACCAAGAAGACATCGATGCCTCAAACATTATAGATGAGGATGTCAGCAGTGGATCCACCATTGAGAAGAGCACCCCAGAAGGCTACATTTTGCACACC
         K  K  G  E  Y  R  T  H  Q  E  D  I  D  A  S  N  I  I  D  E  D  V  S  S  G  S  T  I  E  K  S  T  P  E  G  Y  I  L  H  T

713     GACCTTCCCACTTCACAGCCTACTGGAGACCGGGATGACGCCTTCTTTATTGGGAGCACCCTGGCCACCATTGCAACTACTCCATGGGTTTCTGCCCACACAAAACAGAACCAGGAACGG
         D  L  P  T  S  Q  P  T  G  D  R  D  D  A  F  F  I  G  S  T  L  A  T  I  A  T  T  P  W  V  S  A  H  T  K  Q  N  Q  E  R

833     ACCCAGTGGAACCCGATCCATTCAAACCCAGAAGTACTACTTCAGACAACCACCAGGATGACTGATATAGACAGAAACAGCACCAGTGCTCATGGAGAAAACTGGACCCAGGAACCACAG
         T  Q  W  N  P  I  H  S  N  P  E  V  L  L  Q  T  T  T  R  M  T  D  I  D  R  N  S  T  S  A  H  G  E  N  W  T  Q  E  P  Q

953     CCTCCTTTCAATAACCATGAGTATCAGGATGAAGAGGAGACCCCACATGCTACAAGCACAACCTGGGCAGATCCTAATAGCACAACAGAAGAAGCAGCTACCCAGAAGGAGAAGTGGTTT
         P  P  F  N  N  H  E  Y  Q  D  E  E  E  T  P  H  A  T  S  T  T  W  A  D  P  N  S  T  T  E  E  A  A  T  Q  K  E  K  W  F

1073    GAGAATGAATGGCAGGGGAAGAACCCACCCACCCCAAGTGAAGACTCCCATGTGACAGAAGGGACAACTGCCTCAGCCCACAACAACCATCCAAGTCAAAGAATGACAACACAGAGTCAA
         E  N  E  W  Q  G  K  N  P  P  T  P  S  E  D  S  H  V  T  E  G  T  T  A  S  A  H  N  N  H  P  S  Q  R  M  T  T  Q  S  Q

1193    GAGGATGTTTCATGGACCGATTTCTTCGACCCAATCTCACATCCAATGGGACAAGGTCATCAAACAGAAAGCAAGGGACACTCAAGTGGGAATCAAGACAGTGGAGTGACCACAACTTCT
         E  D  V  S  W  T  D  F  F  D  P  I  S  H  P  M  G  Q  G  H  Q  T  E  S  K  G  H  S  S  G  N  Q  D  S  G  V  T  T  T  S
```

Abb. 2

Totalsequenz der DNA u. des CD44-Proteins von p-Meta-1 (Blatt 1)

......Signalpeptid  ↓——↓ **EZB**  \\\\\\\\\\\\ **Transmembranregion**

1313 GGTCCTGCGAGGAGACCTCAGATTCCAGAGTGGCTTATCATCTTGGCATCCCTCCTGGCGCTGGCTCTGATTCTTGCCGTCTGCATTGCTGTCAACAGTAGGAGAAGGTGTGGGCAGAAG
G P A R R P Q I P E W L I I L A S L L A L A L I L A V C I A V N S R R R C G Q K

1433 AAGAAGCTGGTGATCAACAGTGGCAATGGAACAGTGGAAGACAGGAAACCAAGTGAACTCAACGGGGAGGCCAGCAAGTCTCAGGAAATGGTGCATTTGGTGAACAAGGAACCAACAGAG
K K L V I N S G N G T V E D R K P S E L N G E A S K S Q E M V H L V N K E P T E

1553 ACTCCGGACCAGTTTATGACAGCTGATGAGACCCGGAATCTGCAGAGTGTGGATATGAAGATTGGGGTGTAGTGCCTATGCCACTAACTTGAAAAGACACAACAATTGGAGACATGTCAT
T P D Q F M T A D E T R N L Q S V D M K I G V U

1673 TACTGGGAGCTGGGACCCTTAACAGATGCAATGTGCTACTGATTATTTTTATTGGGATTATTTTGGGCATAAAATTTCCCTTTTTTTGTTTTTTAAAAGTTTGTTTTCCAATTTATGAA

1793 AATAGCATTGCTTTCTGAAATGAGGGTCTCTTCCAGTTCCTCCTTAGAGGCCTTGCATTACCAGGGTATGCTACCATAGGCTTCTACCAAATGAATACTCTTGGTCCCGATTGAACCCAA

1913 AGTCCCAGGTAACATCCACCAGCTAAGGATTTCCCCAGAACTTAGAGAGATTGGTCTCTGGGAGGAAATTTGAATGGGTCCATATTGCCTCCCAGCAGTCCAATCTGTAGGCATTGCTTT

2033 GCAGTGGATGGGAGATCAGGTGTACTGGTTACACACTCTCTTTATAGACTCCCTTCTGCTGGAAAATTTCCACATGCTTCTGAGAGATTCCCCAAAGGTGACGCTATTTATCTTTAGTAA

2153 GCTATTTATCTTTGTTTTTGAAATATCAAACCCTGGAGGTCCTTTTTTCAGTATGACTTTTTTTATTTTGTTTTTTTTTATTTTGTTTTTTAGGTTACTTTGTCAGAAGCATAACAGGGT

2273 ATAAGTTGATTCATAATAAATACCTGTCCATCTTCCATCTTGACCTGTTGTGCTGTGATCCTTCAGTTTCTAAATCAGCAAGGTCTGAGTCTTTGTAGCACATCAATGTGACCTTAGTAT

2393 GGTCCTCTGAAACTCATGTTAGAGCATCCGTGCCCTGCTTGGGTTTACCCAGCTGAATCTCAGAAGATCAAGGACAGGAGCACTGTTTTCATTCTAGGACTATCAAAGGGTTTCTCTCC

2513 TGTTCAAGAATCTGAATTGGGAGTAGGAGAGCTTCTGTCCCTTTTATGTTTCGATAACCACCCATTTCTCTTTCTTAAAGGGCACATTAAGTTTTTATATCTTACAACATTCGCGGTCCT

2633 GTTTCATAGACACTGATCTTATTGGCACTTTCACAAAACAGTGTGGAGGGGACTTCTGACACCTTATAGTAAAAGGAGAAGCCAACAGAAATGAAAGTGTGGACAGAGAGCAGTAGATTG

2753 GCATGAGGAGGCATGATGTACAACCCCCAGACCACTCTTTCCATCACCACATTTGTTGATGCTTTCGCAAGCCAGTTGGTACTTAGAATCAGTTCCCCAGGGAATCCTTCAAAAAGCCAT

2873 AAGAATGCCCACCCCTGGAATCTTACCACCACCAGATGAGCAGGTTTATGGTTTAGCAAAAGGAGAATGCTGTCACCCTCTGACCTCATAGTTTTCACATACTGGGCAAGTGTTCATCTG

2993 CCAGGATGCCCCATTGCTCCTAGGTCTTCCCAGGTACCTTGTAGAAGAACTTAAATCTATAAAATAAGGCTTTCTCTAAAATGGAACTTCCTTTCTAAGGCTCCCATTTTTACTGTTGAC

3113 TAAATTTATATGTTTAATAGTTTTTTTCAAATAAAAACAAACACAAAAAGGAAAAAAAAAAAAAAAAAAAAAAAAAAAAAAAAAAAAAAAAAAA

Abb. 2

Totalsequenz der DNA u. des CD44-Proteins von p-Meta-1 (Blatt 2)

Abb. 3a c-DNA und Proteinsequenz der extracellulären Bereiche

```
                                                                          D I
718  GTACACCCCATCCCAGACGAAGACAGTCCCTGGATCACCGAC---------------AGCACAGACAGAATCCCTGCTACCACAGGCTGGGAGCCAAATGAAGAAAATGAAGATGAAAGA
201   V H P I P D E D S P W I T D - - - - - - S T D R I P A T T G W E P N E E N E D E R   human
205   S Q P T G D R D D A F F I G S T L A T S T E S N T N P T G W K P N E E N E D E T   rat

                                                                  D II
821  GACAGACACCTCAGTTTTTCTGGATCAGGCATTGATGATGATGAAGATTTTATCTCCAGCACCATTTCAACCACACCACGGGCCTTTGACCACACAAAACAGAACCAGGACTGGACCCAG
236   D R H L S F S G S G I D D D E D F I S S T I S T T P R A F D H T K Q N Q D W T Q   human
245   D K Y P N F S G S G I D D D E D F I S S T I A T T P W V S A H T K Q N Q E R T Q   rat

941  TGGAACCCAAGCCATTCAAATCCGGAAGTGCTACTTCAGACAACCACAAGGATGACTGATGTAGACAGAAATGGCACCACTGCTTATGAAGGAAACTGGAACCCAGAAGCACACCCTCCC
276   W N P S H S N P E V L L Q T T T R M T D V D R N G T T A Y E G N W N P E A H P P   human
285   W N P I H S N P E V L L Q T T T R M T D I D R N S T S A H G E N W T Q E P Q P P   rat

                                                     D III
1061 CTCATTCACCATGAGCATCATGAGGAAGAAGAGACCCCACATTCTACAAGCACAATCCAGGCAACTCCTAGTAGTACAACGGAAGAAACAGCTACCCAGAAGGAACAGTGGTTTGGCAAC
316   L I H H E H H E E E E T P H S T S T I Q A T P S S T T E E T A T Q K E Q W F G N   human
325   F N N H E Y Q D E E E T P H A T S T T W A D P N S T T E E A A T Q K E K W F E N   rat

1181 AGATGGCATGAGGGATATCGCCAAACACCCAGAGAAGACTCCCATTCGACAACAGGGACAGCTGCAGCCTCAGCTCATACCAGCCATCCAATGCAAGGAAGGACAACACCAAGCCCAGAG
356   R W H E G Y R Q T P R E D S H S T T G T A A A S A H T S H P M Q G R T T P S P E   human
365   E W Q G K N P P T P S E D S H V T E G T - T A S A H N N H P S Q R M T T Q S Q E   rat

                                                                       D IV
1301 GACAGTTCCTGGACTGATTTCTTCAACCCAATCTCACACCCCATGGGACGAGGTCATCAAGCAGGAAGAAGGATGGATATGGACTCCAGTCATAGTACAACGCTTCAGCCTACTGCAAAT
396   D S S W T D F F N P I S H P M G R G H Q A G R R M D M D S S H S T T L Q P T A N   human
404   D V S W T D F F D P I S H P M G Q G H Q T E S K - D T G S S H S T T L Q P T A A   rat

1421 CCAAACACAGGTTTGGTGGAAGATTTGGACAGGACAGGACCTCTTTCAATGACAACGCAGCAGAGTAATTCTCAGAGCTTCTCTACATCACATGAAGGCTTGGAAGAAGATAAAGACCAT
436   P N T G L V E D L D R T G P L S M T T Q Q S N S Q S F S T S H E G L E E D K D H   human
443   P N T H L V E D L N R T G P L S V T T P Q S H S Q N F S T L P G E L E E G E D H   rat

                                    D V
1541 CCAACAACTTCTACTCTGACATCAAGCAATAGGAATGATGTCACAGGTGGAAGAAGAGACCCAAATCATTCTGAAGGCTCAACTACTTTACTGGAAGGTTATACCTCTCATTACCCACAC
476   P T T S T L T S S N R N D V T G G R R D P N H S E G S T T L L E G Y T S H Y P H   human
483   P T T S V L P S S T K - - - S G R R R G G S L P R D T T T S L E G Y T P Q Y P D   rat

                                                                         end of variant domaine
1661 ACGAAGGAAAGCAGGACCTTCATCCCAGTGACCTCAGCTAAGACTGGGTCCTTTGGAGTTACTGCAGTTACTGTTGGA---GATTCCAACTCTAATGTCAATCGTTCCTTATCAGGAGAC
516   T K E S R T F I P V T S A K T G S F G V T A V T V G - D S N S N V N R S L S G D   human
520   T M E N G T L F P V T P A K T E V F G E T E G T V A T D S N F N V D G S L P G D   rat
```

```
rMeta-1  M D K V W W H T A W G L L C L L Q L S L A Q Q Q I D L N I T C R Y A G V F H V E   40
hCD44    M D K F W W H A A W G L - C L V P L S L A - - Q I D L N I T C R F A G V F H V E   37
mCD44    M D K F W W H T A W G L - C L L Q L S L A H Q Q I D L N V T C R Y A G V F C V E   39

rMeta-1  K N G R Y S I S R T E A A D L C E A F N T T L P T M A Q M E L A L R K G F E T C   80
hCD44    K N G R Y S I S R T E A A D L C K A F N S T L P T M A Q M E K A L S I G F E T C   77
mCD44    K N G R Y S I S R T E A A D L C Q A F N S T L P T M D Q M K L A L S K G F E T C   79

rMeta-1  R Y G F I E G H V V I P R I H P N A I C A A N N T G V Y I L L A S N T S H Y D T  120
hCD44    R Y G F I E G H V V I P R I H P N S I C A A N N T G V Y I L T - Y N T S Q Y D T  116
mCD44    R Y G F I E G N V V I P R I H P N A I C A A N H T G V Y I L V T S N T S H Y D T  119

rMeta-1  Y C F N A S A P L E E D C T S V T D L P N S F D G P V T I T I V N R D G T R Y S  160
hCD44    Y C F N A S A P P E E D C T S V T D L P N A F D G P I T I T I V N R D G T R Y V  156
mCD44    Y C F N A S A P P E E D C T S V T D L P N S F D G P V T I T I V N R D G T R Y S  159

rMeta-1  K K G E Y R T H Q E D I D A S N I I D E D V S S G S T I E K - S T P E G Y I L H  199
hCD44    Q K G E Y R T N P E D I Y P S N P T D D D V S S G S S S E R S S T S G G Y I F Y  196
mCD44    K K G E Y R T H Q E D I D A S N I I D D D V S S G S T I E K - S T P E G Y I L H  198

rMeta-1  T D L P T S Q P T G D R D D A F F I G S T L - - - A T I A T T P W V S A H T K Q  236
hCD44    T F S T - V H P I P D E D S P W I T D S T D R I P A T - - - - - - - - - - - - -  222
mCD44    T Y L P T E Q P T G D Q D D S F F I R S T L - - - A T - - - - - - - - - - - - -  222

rMeta-1  N Q E R T Q W N P I H S N P E V L L Q T T T R M T D I D R N S T S A H G E N W T  276
hCD44    - - - - - - - - - - - - - - - - - - - - - - - - - - - - - - - - - - - - - - - -
mCD44    - - - - - - - - - - - - - - - - - - - - - - - - - - - - - - - - - - - - - - - -

rMeta-1  Q E P Q P P F N N H E Y Q D E E E T P H A T S T T W A D P N S T T E E A A T Q K  316
hCD44    - - - - - - - - - - - - - - - - - - - - - - - - - - - - - - - - - - - - - - - -
mCD44    - - - - - - - - - - - - - - - - - - - - - - - - - - - - - - - - - - - - - - - -

rMeta-1  E K W F E N E W Q G K N P P T P S E D S H V T E G T T A S A H N N H P S Q R M T  356
hCD44    - - - - - - - - - - - - - - - - - - - - - - - - - - - - - - - - - - - - - - - -
mCD44    - - - - - - - - - - - - - - - - - - - - - - - - - - - - - - - - - - - - - - - -

rMeta-1  T Q S Q E D V S W T D F F D P I S H P M G Q G H Q T E S K - - - - - - - - - - -  385
hCD44    - - - - - - - - - - - - - - - - - - - - - - - - - - - - - R D Q D T F H P S - G  232
mCD44    - - - - - - - - - - - - - - - - - - - - - - - - - - - - - R D R D S S K D S R G  233
rCD44    - - - - - - - - - - - - - - - - - - - - - - - - - - - - - S D G D S S M D P R G  234

rMeta-1  - - - - - - - - - - - - G H S S G N Q D S G V T T T S G P A R R P Q I P E W L I  413
hCD44    G S H T - T H E S E S D G H S H G S Q E G G A N T T S G P I R T P Q I P E W L I  271
mCD44    S S R T V T H G S E L A G H S S A N Q D S G V T T T S G P M R R P Q I P E W L I  273
rCD44    G F D T V T H G S E L A                                                          246

rMeta-1  I L A S L L A L A L I L A V C I A V N S R R R C G Q K K K L V I N S G N G T V E  453
hCD44    I L A S L L A L A L I L A V C I A V N S R R R C G Q K K K L V I N S G N G A V E  312
mCD44    I L A S L L A L A L I L A V C I A V N S R R R C G Q K K K L V I N G G N G T V E  313

rMeta-1  D R K P S E L N G E A S K S Q E M V H L V N K E P T E T P D Q F M T A D E T R N  493
hCD44    D R K P S G L N G E A S K S Q E M V H L V N K E S S E T P D Q F M T A D E T R N  352
mCD44    D R K P S E L N G E A S K S Q E M V H L V N K E P S E T P D Q C M T A D E T R N  353

rMeta-1  L Q S V D M K I G V                                                              503
hCD44    L Q N V D M K I G V                                                              362
mCD44    L Q S V D M K I G V                                                              363
```

Abb. 3b

Proteinsequenz von Oberflächenproteinen von Ratten (rMeta-1) Mensch (hCD44) und Maus (mCD44) mit extracellulären Bereichen bei Ratten- und Humanprotein.

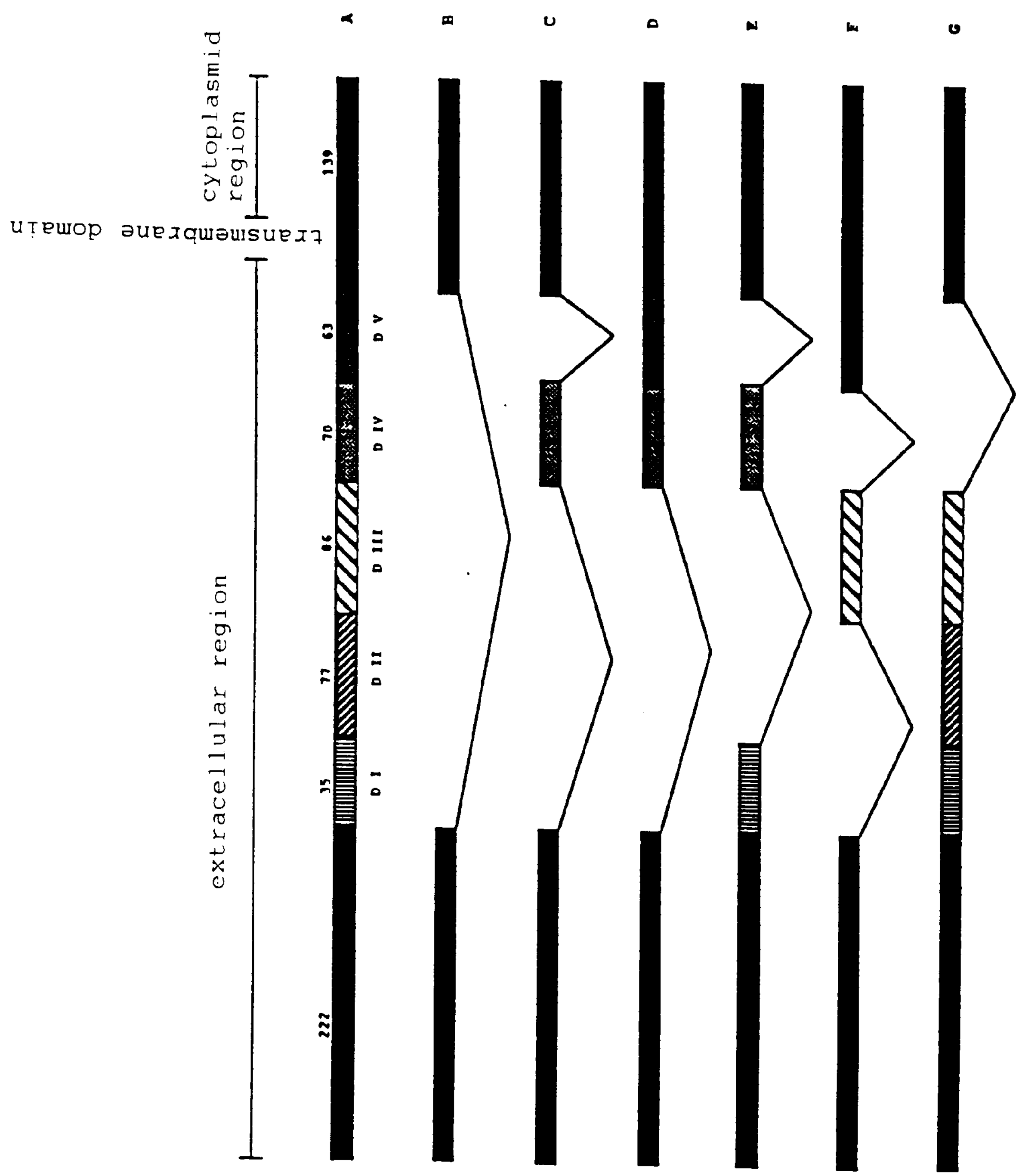
cytoplasmid region
transmembrane domain
extracellular region
222
35
77
86
70
63
139
D I
D II
D III
D IV
D V
A
B
C
D
E
F
G

Fig.4

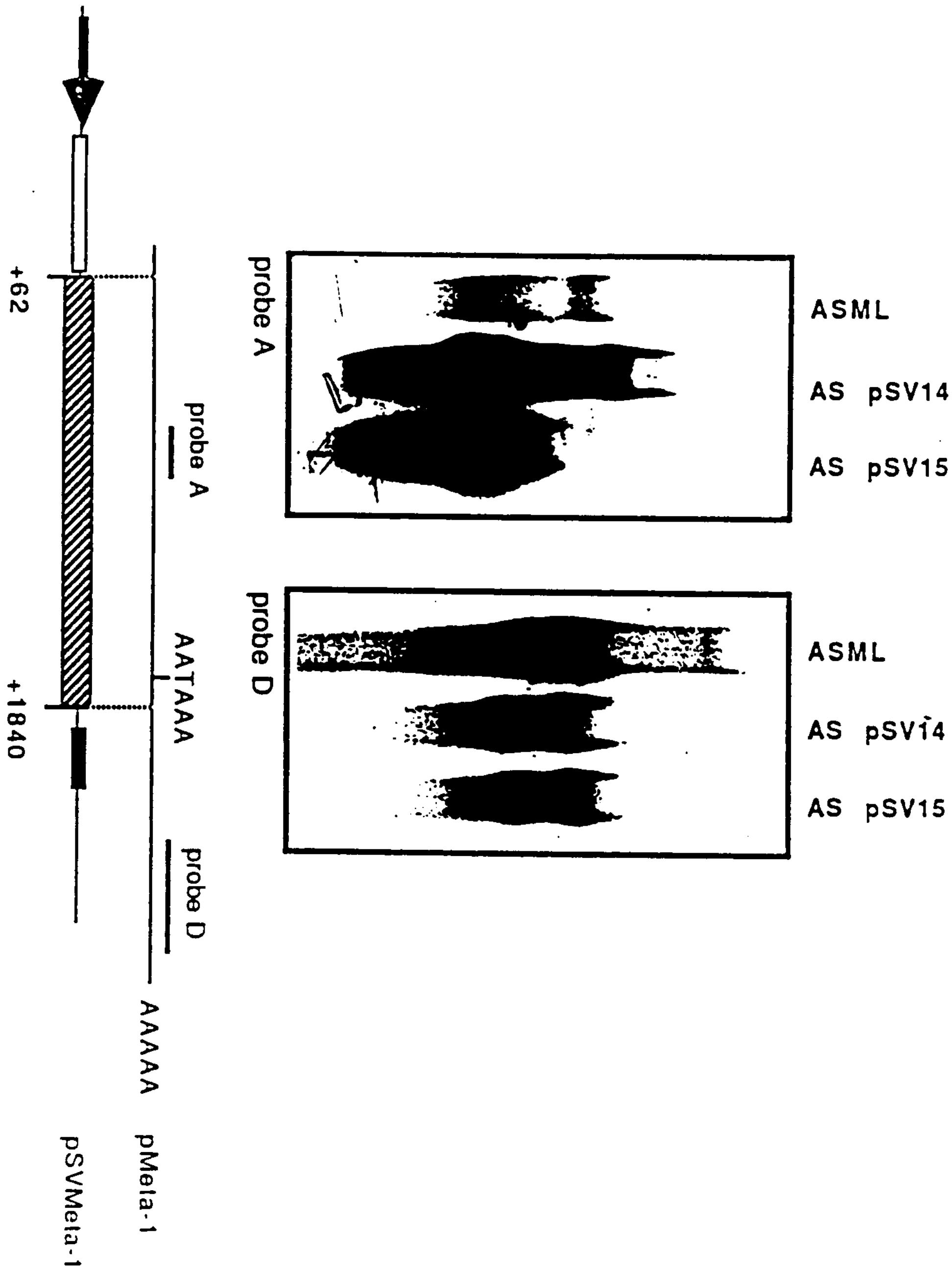
+62
+1840
probe A
AATAAA
probe D
AAAAA
pMeta-1
pSVMeta-1
probe A
ASML
AS pSV14
AS pSV15
probe D
ASML
AS pSV14
AS pSV15

Fig. 5